(19) Europäisches Patentamt / European Patent Office / Office européen des brevets

(11) **EP 4 613 722 A1**

(12) **EUROPEAN PATENT APPLICATION**
published in accordance with Art. 153(4) EPC

(43) Date of publication:
**10.09.2025 Bulletin 2025/37**

(21) Application number: **23885856.7**

(22) Date of filing: **02.11.2023**

(51) International Patent Classification (IPC):
**C04B 35/486** (2006.01)    **A61C 13/083** (2006.01)
**A61K 6/818** (2020.01)      **A61K 6/824** (2020.01)
**A61L 27/10** (2006.01)      **C01G 25/00** (2006.01)
**C01G 25/02** (2006.01)

(52) Cooperative Patent Classification (CPC):
**A61C 13/083; A61K 6/818; A61K 6/824;**
**A61L 27/10; C01G 25/00; C01G 25/02;**
**C04B 35/486**

(86) International application number:
**PCT/JP2023/039628**

(87) International publication number:
**WO 2024/096102 (10.05.2024 Gazette 2024/19)**

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**KH MA MD TN**

(30) Priority: **02.11.2022 JP 2022176790**
**26.12.2022 JP 2022208405**

(71) Applicant: **Kuraray Noritake Dental Inc.**
**Kurashiki-shi, Okayama 710-0801 (JP)**

(72) Inventors:
• **NAKANO, Kirihiro**
  **Miyoshi-shi, Aichi 470-0293 (JP)**
• **KASHIKI, Nobusuke**
  **Miyoshi-shi, Aichi 470-0293 (JP)**
• **NIWA, Takahiro**
  **Miyoshi-shi, Aichi 470-0293 (JP)**

(74) Representative: **D Young & Co LLP**
**3 Noble Street**
**London EC2V 7BQ (GB)**

(54) **ZIRCONIA PRE-SINTERED BODY, COMPOSITION, AND METHODS FOR PRODUCING SAME**

(57)    The present invention provides pre-sintered bodies, compositions, and methods for their production that enable the production of zirconia sintered bodies that can maintain high translucency comparable to that achieved by extended sintering, even after short sintering with a hold time of 10 minutes or less at the highest sintering temperature. The present invention relates to a zirconia pre-sintered body with zirconia particles having consolidated to an extent not reaching full sintering,
the zirconia pre-sintered body comprising a stabilizer capable of preventing a phase transformation of zirconia, and satisfying the formula (1) below when $\Delta L_1{}^*$(W-B) related to a first sintered body fabricated by sintering at 1,550°C for 120 minutes is compared to $\Delta L_2{}^*$(W-B) related to a second sintered body fabricated by sintering at 1,550°C for 10 minutes,

$$\Delta L_2{}^*\text{(W-B)} / \Delta L_1{}^*\text{(W-B)} \geq 0.85 \qquad (1).$$

# EP 4 613 722 A1

## Description

### TECHNICAL FIELD

The present invention relates to zirconia pre-sintered bodies, compositions, and methods for their production. More specifically, the present invention relates to zirconia (zirconium(IV) oxide or $ZrO_2$) pre-sintered bodies and compositions that possess high translucency even after short sintering, and to methods of production thereof.

### BACKGROUND ART

Oxide ceramics find wide industrial applications. Among such oxide ceramics are zirconia sintered bodies containing yttria, which, due to their high strength and aesthetic qualities, have found use in dental material applications, such as dental prostheses. Typically, the primary raw materials for these zirconia sintered bodies are either individual zirconium oxide powder and yttrium oxide powder or their solid solution. These raw materials are pulverized and mixed, followed by drying, shaping, and heat treatment to yield a pre-sintered body (mill blank). This pre-sintered body is then machined into a form resembling the intended dental prosthesis, and subsequently sintered to create a dental material such as a dental prosthesis.

In sintering of dental prostheses, shorter sintering times enable shorter treatments. What is also important is that the prosthetic's aesthetic quality remains consistent regardless of variations in the output of the sintering furnace. In other words, it is important to maintain consistent translucency across a range of sintering durations, whether short or extended.

One proposed method for producing zirconia sintered bodies is described in Patent Literature 1, for example. The method of production disclosed in Patent Literature 1 produces a zirconia sintered body that is a mixture of a tetragonal solid solution of zirconia and yttria and a cubic solid solution of zirconia and yttria.

The sintering of zirconia involves energy stabilization, accompanied by mass transfer that induces changes in shape and crystal phase. Consequently, the method of

Patent Literature 1 faces challenges when, for example, sintering is terminated after a short period, such as inadequate densification causing poor transparency, or insufficient crystal phase transformation and/or crystallization, resulting in diminished strength and/or transparency.

Proposals have been made to solve this issue involving short sintering time, as described in Patent Literatures 2 and 3, for example. Patent Literature 2 discloses using zirconium oxide and yttrium oxide individually as raw materials along with a tetragonal solid solution of zirconium oxide and yttrium oxide. It states that this reduces the translucency difference between zirconia sintered bodies produced with short and extended retention periods-one with a hold time of 30 minutes and the other with a longer hold time of approximately 2 hours at the highest sintering temperature. Patent Literature 2 also discloses that excellent translucency can be achieved with a sintering time of 15 minutes.

Patent Literature 3 discloses a dental zirconia machinable body for cutting. This zirconia machinable body, featuring a porosity of 15 to 30%, uses materials such as Zpex Smile® as powder material or machinable body for the production of sintered bodies. A specific embodiment discloses that a sintered body with excellent translucency and strength was obtained with short sintering.

### CITATION LIST

Patent Literature

Patent Literature 1: JP 2018-52806 A
Patent Literature 2: WO2018/056330
Patent Literature 3: JP 2020-033338 A

### SUMMARY OF INVENTION

Technical Problem

Shorter sintering times offer advantages in reducing manufacturing costs. After investigation, the present inventors have found that the invention according to Patent Literature 1 presents the issue of low translucency when the hold time at the highest sintering temperature is reduced from approximately 30 minutes to as short as, for example, 10 minutes to achieve an even shorter sintering time, as compared to a longer retention period of, for example, about 2 hours, due to an insufficient concentration gradient of zirconium elements and yttrium elements to facilitate sintering through mass transfer between tetragonal and cubic crystals.

Patent Literature 2 also involves the issue of low translucency. When the hold time at the highest sintering temperature is reduced to 10 minutes, a high fraction of monoclinic system remains in the pre-sintered body without undergoing phase transformation, and the reaction rate is insufficient for the phase transformation to occur from the monoclinic system to tetragonal or cubic system, leading to low translucency compared to a longer retention period with a hold time of approximately 2 hours at the highest sintering temperature, as opposed to when the retention time is short with a hold time of, for example, about 10 minutes.

In Patent Literature 3, there is no mention of using undissolved yttria in short sintering with a hold time of 2 minutes at the highest sintering temperature. Instead, it merely discloses zirconia machinable bodies using a solid solution of yttrium in zirconia. In Reference Example 1, it is stated that sufficient translucency cannot be achieved without yttrium dissolved in zirconia as a solid solution. Regarding short sintering, it is not explicitly stated that the same level of translucency can be achieved when the zirconia sintered body is produced by short sintering with a 2-minute hold time and when it is produced with a hold time of 2 hours.

In Patent Literature 3, short sintering cannot be achieved with yttrium compounds that do not form a solid solution with zirconia. This is because such yttrium compounds do not favorably induce material diffusion.

Consequently, when the zirconia pre-sintered body contains yttrium compounds that do not form a solid solution with zirconia, the zirconia sintered body shows a low translucency when, for example, it is produced with a short retention period with a hold time of approximately 10 minutes at the highest sintering temperature, as compared to a longer retention period with a hold time of about 2 hours.

It is an object of the present invention to provide zirconia pre-sintered bodies, compositions, and methods for their production that enable the production of zirconia sintered bodies that can maintain high translucency comparable to that achieved by extended sintering, even after short sintering with a hold time of 10 minutes or less at the highest sintering temperature.

Solution to Problem

The present inventors conducted intensive studies to find a solution to the foregoing issues, and found that, in zirconia pre-sintered bodies (mill blank), controlling the range of average primary particle diameters of particles constituting the raw material powders used for the production of pre-sintered bodies, as well as the level of the concentration gradient of zirconium and yttrium elements between crystals within these particles-specifically, the standard deviation range of yttrium element distribution at the microscopic level-is advantageous in favorably facilitating material diffusion and obtaining zirconia sintered bodies that can maintain high translucency comparable to that achieved by extended sintering, even after short sintering. Building upon this finding, the present inventors conducted additional examinations, ultimately resulting in the completion of the present invention.

Specifically, the present invention includes the following.

[1] A zirconia pre-sintered body with zirconia particles having consolidated to an extent not reaching full sintering, the zirconia pre-sintered body comprising zirconia, and a stabilizer capable of preventing a phase transformation of zirconia, and satisfying the formula (1) below when $\Delta L_1^*$(W-B) related to a first sintered body fabricated by sintering the zirconia pre-sintered body at 1,550°C for 120 minutes is compared to $\Delta L_2^*$(W-B) related to a second sintered body fabricated by sintering the zirconia pre-sintered body at 1,550°C for 10 minutes,

$$\Delta L_2^*(W\text{-}B) \,/\, \Delta L_1^*(W\text{-}B) \geq 0.85 \qquad (1).$$

[2] The zirconia pre-sintered body according to [1], which satisfies the following condition (i) or (ii),

(i) the zirconia comprises tetragonal zirconia, and a part of the stabilizer is not dissolved in zirconia as a solid solution;
(ii) the zirconia comprises monoclinic zirconia and cubic zirconia.

[3] The zirconia pre-sintered body according to [1] or [2], wherein the stabilizer is yttria ($Y_2O_3$).
[4] The zirconia pre-sintered body according to [3], which satisfies any of the following conditions (A-1), (A-2), (A-3), (A-4), (A-5), and (A-6),

(A-1) the zirconia comprises monoclinic zirconia and tetragonal zirconia, where the monoclinic content is 55% or more and the tetragonal content is 10% or more, and a part of the yttria is not dissolved in zirconia as a solid solution;

(A-2) the zirconia comprises tetragonal zirconia, and, without corresponding to (A-1), the tetragonal content is 10% or more, and a part of the yttria is not dissolved in zirconia as a solid solution;

(A-3) the zirconia comprises monoclinic zirconia and cubic zirconia, where the monoclinic content is 55% or more, and the cubic content is 15% or more;

(A-4) the zirconia comprises monoclinic zirconia and cubic zirconia, and, without corresponding to (A-3), the cubic content is 15% or more;

(A-5) the zirconia comprises tetragonal zirconia and cubic zirconia, where the tetragonal content is 5% or more and less than 50%, and the cubic content is 50% or more and less than 95%, and a part of the yttria is not dissolved in zirconia as a solid solution;

(A-6) the zirconia comprises tetragonal zirconia and cubic zirconia, and, without corresponding to (A-5), the cubic content is 10% or more and less than 50%, and a part of the yttria is not dissolved in zirconia as a solid solution, the tetragonal content, cubic content, and monoclinic content in the above (A-1), (A-2), (A-3), (A-4), (A-5), or (A-6) being calculated from the following formulae, respectively,

$$\text{tetragonal fraction } f_t \ (\%) = I_t \ / \ (I_m + I_t + I_c + I_y) \times 100 \qquad (2\text{-}1)$$

$$\text{cubic fraction } f_c \ (\%) = I_c \ / \ (I_m + I_t + I_c + I_y) \times 100 \qquad (2\text{-}2)$$

$$\text{monoclinic fraction } f_m \ (\%) = I_m \ / \ (I_m + I_t + I_c + I_y) \times 100 \qquad (2\text{-}3),$$

wherein $f_m$, $f_t$, and $f_c$ represent the monoclinic fraction (%), tetragonal fraction (%), and cubic fraction (%), respectively, $I_m$ represents the peak area intensity near $2\theta = 28.2°$, where the peak top of the main peak of the monoclinic system appears in XRD measurement, It represents the peak area intensity near $2\theta = 30.2°$, where the peak top of the main peak of the tetragonal system appears in XRD measurement, $I_c$ represents the peak area intensity near $2\theta = 30.1°$, where the peak top of the main peak of the cubic system appears in XRD measurement, and $I_y$ represents the peak area intensity near $2\theta = 29.2°$, where the peak top of the main peak of undissolved yttria appears in XRD measurement.

[5] The zirconia pre-sintered body according to [3] or [4], wherein the standard deviation of yttrium element distribution is 2 mol% or more and less than 21 mol%.

[6] The zirconia pre-sintered body according to [4] or [5], which satisfies the condition (A-1) or (A-2), and in which the proportion of yttria not dissolved in zirconia as a solid solution is 1 to 25%.

[7] The zirconia pre-sintered body according to [4] or [5], which satisfies the condition (A-3) or (A-4), and in which a part of the yttria is not dissolved in zirconia as a solid solution, and the proportion of the yttria not dissolved in zirconia as a solid solution is 1 to 15%.

[8] The zirconia pre-sintered body according to any one of [1] to [7], wherein the content of the stabilizer is 2 to 9 mol% relative to the total mole of the zirconia and the stabilizer.

[9] The zirconia pre-sintered body according to any one of [1] to [8], which has a density of 3.6 g/cm$^3$ or less.

[10] The zirconia pre-sintered body according to any one of [1] to [9], which has an average primary particle diameter of 40 to 110 nm.

[11] A zirconia composition comprising zirconia, and a stabilizer capable of preventing a phase transformation of zirconia, and satisfying the formula (1) below when $\Delta L_1{}^*(W\text{-}B)$ related to a first sintered body fabricated by sintering the zirconia composition at 1,550°C for 120 minutes is compared to $\Delta L_2{}^*(W\text{-}B)$ related to a second sintered body fabricated by sintering the zirconia composition at 1,550°C for 10 minutes,

$$\Delta L_2{}^*(W\text{-}B) \ / \ \Delta L_1{}^*(W\text{-}B) \geq 0.85 \qquad (1).$$

[12] The zirconia composition according to [11], which satisfies the following condition (i) or (ii),

(i) the zirconia comprises a tetragonal zirconia powder (T), and a part of the stabilizer is a stabilizer powder that is not dissolved in zirconia as a solid solution;

(ii) the zirconia comprises a monoclinic zirconia powder (M) and a cubic zirconia powder (C).

[13] The zirconia composition according to [12], which satisfies the condition (i), and further comprises a monoclinic zirconia powder (M).

[14] The zirconia composition according to [13], wherein the ratio of the total mass of the zirconia powder (T) and the zirconia powder (M) to the mass of the stabilizer powder is 85.0 mass%:15.0 mass% to 99.8 mass%:0.2 mass%.

[15] The zirconia composition according to [13] or [14], wherein the zirconia powder (M) is 0 to 85 mass% of the total mass of the zirconia powder (T) and the zirconia powder (M).

[16] The zirconia composition according to any one of [12] to [15], wherein the zirconia powder (T) comprises a dissolved stabilizer capable of preventing a phase transformation of zirconia, and the content of the dissolved stabilizer is 2 to 4 mol% relative to the total mole of the zirconia and the stabilizer.

[17] The zirconia composition according to [12], which satisfies the condition (ii), and in which a part of the stabilizer is not dissolved in zirconia as a solid solution.

[18] The zirconia composition according to [12] or [17], wherein the ratio of the total mass of the zirconia powder (M) and the zirconia powder (C) to the mass of the stabilizer powder is 85.0 mass%:15.0 mass% to 100 mass%:0 mass%.

[19] The zirconia composition according to [12], [17], or [18], wherein the zirconia powder (C) is 15.0 to 95.0 mass% of the total mass of the zirconia powder (M) and the zirconia powder (C).

[20] The zirconia composition according to any one of [12] , [17] to [19], wherein the content of the stabilizer in the zirconia powder (M) is 0 to 1 mol% relative to the total mole of the zirconia powder (M) and the stabilizer.

[21] The zirconia composition according to any one of [12], [17] to [20], wherein the zirconia powder (C) comprises a dissolved stabilizer capable of preventing a phase transformation of zirconia, and the content of the dissolved stabilizer is 5 mol% or more and 15 mol% or less relative to the total mole of the zirconia powder (C) and the stabilizer.

[22] The zirconia composition according to [12], which satisfies the condition (i), and further comprises a cubic zirconia powder (T), wherein the ratio of the total mass of the zirconia powder (T) and the zirconia powder (C) to the mass of the stabilizer powder is 88.0 mass%:12.0 mass% to 99.999 mass%:0.001 mass%.

[23] The zirconia composition according to any one of [11] to [22], wherein the zirconia powder (T), the zirconia powder (C), and the zirconia powder (M) each have an average primary particle diameter (r1) of 40 to 110 nm.

[24] A method for producing a zirconia pre-sintered body, comprising firing the zirconia compositions of [11] to [23] to such an extent that zirconia particles do not fully sinter.

[25] A method for producing a zirconia sintered body, comprising sintering the zirconia pre-sintered bodies of [1] to [10].

Advantageous Effects of Invention

With the zirconia pre-sintered bodies, compositions, and methods for their production of the present invention, zirconia sintered bodies can be obtained that can maintain high translucency comparable to that achieved by extended sintering, even after short sintering with a hold time of 10 minutes or less at the highest sintering temperature.

With the zirconia pre-sintered bodies, compositions, and methods for their production of the present invention, it is also possible to obtain zirconia sintered bodies that can maintain high translucency comparable to that achieved by extended sintering, even after short sintering with a highest sintering temperature of less than 1,600°C (particularly preferably, 1,560°C or less) and a hold time of 10 minutes or less at the highest sintering temperature. This offers excellent production efficiency, making the invention industrially advantageous.

DESCRIPTION OF EMBODIMENTS

A zirconia pre-sintered body of the present invention comprises zirconia, and a stabilizer capable of preventing a phase transformation of zirconia, and satisfies the formula (1) below when $\Delta L_1^*(W-B)$ related to a first sintered body fabricated by sintering at 1,550°C for 120 minutes is compared to $\Delta L_2^*(W-B)$ related to a second sintered body fabricated by sintering at 1,550°C for 10 minutes,

$$\Delta L_2^*(W-B) / \Delta L_1^*(W-B) \geq 0.85 \qquad (1).$$

The zirconia pre-sintered body refers to a material that can be a precursor (intermediate product) of a zirconia sintered body. In this specification, a zirconia pre-sintered body refers to a material in which zirconia particles have consolidated to an extent not reaching full sintering.

The phrase "to an extent not reaching full sintering" refers to a state where complete sintering has not been achieved (semi-sintered state). In a fully sintered state, a zirconia sintered body has undergone densification accompanied by an increase in relative density due to sintering, and exhibits a relative density of 95% or more. The relative density can be calculated as a ratio of the actual density, measured by the Archimedes method, with respect to the theoretical density.

In this specification, the upper limits and lower limits of numeric ranges (for example, ranges of contents of components, ranges of values calculated from components, and ranges of physical properties) can be appropriately combined.

In this specification, the total amount of tetragonal fraction $f_t$, cubic fraction $f_c$, monoclinic fraction $f_m$, and undissolved yttria fraction $f_y$, calculated from the formulae (2-1), (2-2), (2-3), and (2-4) below, does not exceed 100%.

[Zirconia Pre-Sintered Body]

A zirconia pre-sintered body of the present invention comprises zirconia, and a stabilizer capable of preventing a phase transformation of zirconia (hereinafter, also referred to simply as "stabilizer").

Examples of the stabilizer include oxides such as calcium oxide (CaO), magnesium oxide (MgO), yttrium oxide ($Y_2O_3$; hereinafter, also referred to as "yttria"), cerium oxide ($CeO_2$), scandium oxide ($Sc_2O_3$), niobium oxide ($Nb_2O_5$), lanthanum oxide ($La_2O_3$), erbium oxide ($Er_2O_3$), praseodymium oxide ($Pr_2O_3$, $Pr_6O_{11}$), samarium oxide ($Sm_2O_3$), europium oxide ($Eu_2O_3$), thulium oxide ($Tm_2O_3$), gallium oxide ($Ga_2O_3$), indium oxide ($In_2O_3$), and ytterbium oxide ($Yb_2O_3$). Preferred is yttria. The stabilizer may be incorporated alone, or two or more thereof may be incorporated in combination.

The content of the stabilizer in a zirconia pre-sintered body of the present invention is preferably 2 mol% or more, more preferably 3 mol% or more, even more preferably 3.5 mol% or more, particularly preferably 4 mol% or more relative to the total mole of zirconia (zirconium(IV) oxide or $ZrO_2$) and stabilizer. A stabilizer content of 2 mol% or more is preferable because it increases the proportion of the cubic crystal form within the zirconia sintered body, thereby enhancing translucency.

In view of reducing a decrease in the strength of the sintered body, the stabilizer content is preferably 9 mol% or less, more preferably 8 mol% or less, even more preferably 7.5 mol% or less, particularly preferably 7 mol% or less.

The stabilizer content may fall in any combinations of these ranges. For example, the stabilizer content is preferably 2 to 9 mol%, more preferably 3 to 8 mol%, even more preferably 3.5 to 7.5 mol%, particularly preferably 4 to 7 mol%.

The stabilizer content in a zirconia pre-sintered body of the present invention can be adjusted by, for example, making adjustments to the undissolved stabilizer content in the zirconia composition described later, or changing the composition ratio while giving consideration to the content of the dissolved stabilizer in each crystal system, such as by adjusting the content of cubic zirconia, which contains a high proportion of stabilizer dissolved in zirconia as a solid solution.

In this specification, the stabilizer content means the total content of the stabilizer forming a solid solution with zirconia, and the stabilizer not dissolved in zirconia as a solid solution.

The stabilizer content in a zirconia pre-sintered body of the present invention can be measured using techniques, for example, such as inductively coupled plasma (ICP) emission spectral analysis, and X-ray fluorescence analysis (XRF).

A zirconia pre-sintered body of the present invention satisfies the formula (1) below when $\Delta L_1^*(W\text{-}B)$ related to a first sintered body fabricated by sintering at 1,550°C for 120 minutes is compared to $\Delta L_2^*(W\text{-}B)$ related to a second sintered body fabricated by sintering at 1,550°C for 10 minutes,

$$\Delta L_2^*(W\text{-}B) \, / \, \Delta L_1^*(W\text{-}B) \geq 0.85 \qquad (1).$$

Both $\Delta L_1^*(W\text{-}B)$ related to the first sintered body and $\Delta L_2^*(W\text{-}B)$ related to the second sintered body represent values calculated using the L* value for lightness (color space) in the L*a*b* color system (JIS Z 8781-4:2013, Color Measurements - Part 4: CIE 1976 L*a*b* color space). The lightness value L* can be measured with a spectrophotometer, for example, such as the Crystaleye manufactured by Olympus Corporation under this trade name, or the CM-3610A or CM-36dGV manufactured by Konica Minolta Inc., using a D65 illuminant.

Both $\Delta L_1^*(W\text{-}B)$ related to the first sintered body and $\Delta L_2^*(W\text{-}B)$ related to the second sintered body are derived by chromaticity measurement where the lightness (LW*) against a white background and the lightness (LB*) against a black background are measured for the same sample (zirconia sintered body) using the same measurement device, measurement mode, and light source. The difference between these values ($\Delta L^* = (LW^*) - (LB^*)$) then represents an indicator of translucency.

In the evaluation of $\Delta L_1^*(W\text{-}B)$ related to the first sintered body and $\Delta L_2^*(W\text{-}B)$ related to the second sintered body, the highest sintering temperature is 1,550°C, with the rate of temperature increase and the rate of temperature decrease maintained under the same conditions.

In the evaluation of $\Delta L_1^*(W\text{-}B)$ related to the first sintered body, the hold time (retention time) at the highest sintering temperature is 120 minutes (hereinafter, sintering with a hold time of 120 minutes at the highest sintering temperature will also be referred to as "120-minute sintering" or "extended sintering").

In the evaluation of $\Delta L_2^*(W\text{-}B)$ related to the second sintered body, the hold time (retention time) at the highest sintering temperature is 10 minutes (hereinafter, firing with a hold time of 10 minutes at the highest sintering temperature will also be referred to as "10-minute sintering" or "short sintering").

A zirconia pre-sintered body of the present invention can yield a zirconia sintered body that maintains high translucency comparable to that achieved by extended sintering, even after short sintering with a hold time of 10 minutes or less at the highest sintering temperature. A possible explanation for this is as follows.

While the following explanation will be given through the case where the stabilizer is yttria, the present invention is not restricted to cases where the stabilizer is yttria.

Typically, when the hold time at the highest sintering temperature is as short as 10 minutes, there is hardly any movement of yttrium elements or yttrium ions between materials if the stabilizer has a low concentration gradient of its metal elements (preferably, yttrium element) in the crystal system of zirconia. This leads to inadequate stabilization of energy due to mass transfer, resulting in insufficient sintering and a decrease in translucency, presenting challenges associated with 10-minute sintering.

In contrast, as described in the embodiments corresponding to condition (i) or (ii) below (for example, such as condition (A-1), (A-2), (A-3), (A-4), (A-5), or (A-6)), a source of yttrium elements or yttrium ions and the destination for their movement will be present within the system of zirconia pre-sintered body when the pre-sintered body is formed by selecting a zirconia powder of a specific crystal system along with a yttria powder according to conditions such as the amount of dissolved yttria. This results in an appropriate concentration gradient of yttrium elements or yttrium ions between the two, allowing for the movement of yttrium elements or yttrium ions during sintering between the crystals of the zirconia crystal system.

In this way, by ensuring that the system of zirconia pre-sintered body contains both a crystal system of zirconia or a yttrium source, which provides yttrium elements or yttrium ions, and a crystal system of zirconia as the destination for the movement of yttrium elements or yttrium ions, a greater concentration difference of yttrium elements or yttrium ions can be achieved during sintering between materials within the system. Because mass transfer takes place as the particles consolidate during sintering, the movement of yttrium elements or yttrium ions proceeds sufficiently even with a short sintering time of 10 minutes or less, and, through the combined action with the raw material powder having a predetermined average particle diameter, the resulting zirconia sintered body can achieve a more uniform distribution of yttrium elements, satisfying the formula (1) above regarding its translucency.

Patent Literature 3 discloses an example achieving a hold time as short as 2 minutes at the highest sintering temperature, only with a zirconia powder containing 4 mol% or more and 5.5 mol% or less of yttrium dissolved in zirconia as a solid solution.

However, in Patent Literature 3, the example achieving a reduced hold time does not involve dispersing undissolved yttrium compounds on the outermost surface of zirconia particles, as seen in other examples. Instead, it uses only yttrium dissolved in zirconia as a solid solution. Furthermore, the reduced hold time is achieved by the production process where CIP is performed multiple times (specifically, 5 to 10 times) to reduce the porosity of the machinable body, and sintering is conducted at high temperatures with the highest sintering temperature of 1,600°C. Considering industrial usefulness, the process requiring repeated CIP processing (5 times or more) to obtain a machinable body, and a high-temperature treatment with the highest sintering temperature of 1,600°C suffers from poor production efficiency, and is economically disadvantageous in industrial applications, leaving room for further improvement.

In contrast, the present invention facilitates mass transfer during sintering by increasing the concentration difference of yttrium elements or yttrium ions between materials within the system being sintered, allowing for superior translucency with a short sintering time of 10 minutes or less, even when the highest sintering temperature is below 1,600°C, as described above. Consequently, unlike Patent Literature 3, repeated CIP processing (5 times or more) is not necessary in producing the molded body. By allowing sintering to proceed with a lower highest sintering temperature in a shorter period, the present invention achieves a reduction in both highest sintering temperature and sintering time, offering industrial advantages.

The present invention can exhibit its effects as long as the movement of yttrium elements or yttrium ions is facilitated, even in crystal systems different from the one described in Patent Literature 3 (for example, even when the monoclinic content of the zirconia in the zirconia pre-sintered body is 55% or more).

As described above, the present invention can demonstrate its effectiveness even with yttria that is not dissolved in zirconia as a solid solution (hereinafter, also referred to as "undissolved yttria"). This provides an advantage over Patent Literature 3, where Reference Example 1 indicates that the amount of dissolved yttrium will be deficient unless 4 mol% or more of dissolved yttrium is used, and short sintering fails to promote sufficient phase transformation, resulting in inadequate translucency.

The following describes a preferred embodiment where the stabilizer is yttria.

The present invention, however, is not restricted to the use of yttria as the stabilizer. Accordingly, when other stabilizers are used, "yttria content" can be read as referring to the content of the respective stabilizers, and "yttrium element distribution" as the element distribution of other metal elements.

In view of more easily facilitating the movement of yttrium elements or yttrium ions in a short sintering period and yielding a zirconia sintered body with superior translucency, it is preferable in a zirconia pre-sintered body of the present invention that the standard deviation of the yttrium element distribution be 2 mol% or more. In view of even superior translucency, the standard deviation of the yttrium element distribution is more preferably 2.1 mol% or more, even more preferably 2.5 mol% or more, particularly preferably 3 mol% or more.

In view of more easily facilitating the movement of yttrium elements or yttrium ions between materials within the system being sintered during short sintering and yielding a zirconia sintered body with even superior translucency, the standard deviation of the yttrium element distribution is preferably less than 21 mol%, more preferably 20 mol% or less, even more preferably 18 mol% or less, particularly preferably 15 mol% or less.

The standard deviation of the yttrium element distribution may fall in any combinations of these ranges. For example, the standard deviation of the yttrium element distribution is preferably 2 mol% or more and less than 21 mol%, more preferably 2.1 mol% or more and 20 mol% or less, even more preferably 2.5 mol% or more and 18 mol% or less, particularly preferably 3 mol% or more and 15 mol% or less.

A certain preferred embodiment is, for example, a zirconia pre-sintered body in which the standard deviation of the yttrium element distribution is 2.1 mol% or more and 15 mol% or less.

The standard deviation of the yttrium element distribution can be adjusted according to factors such as undissolved yttria fraction $f_y$, the predetermined average particle diameter of the raw material powder used as a yttrium source, the content of the predetermined raw material powder in the raw material composition, and the mixing ratio. Notably, adjustments can be more easily achieved by varying the undissolved yttria fraction $f_y$, and the predetermined average particle diameter of the raw material powder used as a yttrium source.

The method of calculation of the standard deviation of the yttrium element distribution is as described in the EXAMPLES section below.

In view of more easily facilitating the movement of yttrium elements or yttrium ions in a short sintering period and yielding a zirconia sintered body with superior translucency, a zirconia pre-sintered body of the present invention is preferably one satisfying the following condition (i) or (ii).

(i) the zirconia comprises tetragonal zirconia, and a part of the stabilizer is not dissolved in zirconia as a solid solution
(ii) the zirconia comprises monoclinic zirconia and cubic zirconia

Examples of embodiments satisfying the condition (i) include:

a zirconia pre-sintered body in which the zirconia consists of tetragonal zirconia, and a part of the stabilizer is not dissolved in zirconia as a solid solution;
a zirconia pre-sintered body in which the zirconia comprises monoclinic zirconia and tetragonal zirconia, and a part of the stabilizer is not dissolved in zirconia as a solid solution; and
a zirconia pre-sintered body in which the zirconia comprises tetragonal zirconia and cubic zirconia, and a part of the stabilizer is not dissolved in zirconia as a solid solution.

A specific preferred embodiment satisfying the condition (i) is a zirconia pre-sintered body satisfying the condition (A-1) or (A-2), or the condition (A-5) or (A-6) below.

A specific preferred embodiment satisfying the condition (ii) is a zirconia pre-sintered body satisfying the condition (A-3) or (A-4) below.

In any of the embodiments, variables such as the yttria content, and the standard deviation of the yttrium element distribution can be selected in appropriate combinations within the ranges provided in this specification.

(A-1) the zirconia comprises monoclinic zirconia and tetragonal zirconia, where the monoclinic content is 55% or more and the tetragonal content is 10% or more, and a part of the yttria is not dissolved in zirconia as a solid solution;
(A-2) the zirconia comprises tetragonal zirconia, and, without corresponding to (A-1), the tetragonal content is 10% or more, and a part of the yttria is not dissolved in zirconia as a solid solution;
(A-3) the zirconia comprises monoclinic zirconia and cubic zirconia, where the monoclinic content is 55% or more, and the cubic content is 15% or more;
(A-4) the zirconia comprises monoclinic zirconia and cubic zirconia, and, without corresponding to (A-3), the cubic content is 15% or more;
(A-5) the zirconia comprises tetragonal zirconia and cubic zirconia, where the tetragonal content is 5% or more and less than 50%, and the cubic content is 50% or more and less than 95%, and a part of the yttria is not dissolved in zirconia as a solid solution;
(A-6) the zirconia comprises tetragonal zirconia and cubic zirconia, and, without corresponding to (A-5), the cubic content is 10% or more and less than 50%, and a part of the yttria is not dissolved in zirconia as a solid solution,
the tetragonal content, cubic content, and monoclinic content in the above (A-1), (A-2), (A-3), (A-4), (A-5), or (A-6) being calculated from the following formulae, respectively,

$$\text{tetragonal fraction } f_t \ (\%) = I_t / (I_m + I_t + I_c + I_y) \times 100 \qquad (2\text{-}1)$$

$$\text{cubic fraction } f_c \ (\%) = I_c / (I_m + I_t + I_c + I_y) \times 100 \qquad (2\text{-}2)$$

$$\text{monoclinic fraction } f_m \ (\%) = I_m / (I_m + I_t + I_c + I_y) \times 100 \qquad (2\text{-}3),$$

wherein $f_m$, $f_t$, and $f_c$ represent the monoclinic fraction (%), tetragonal fraction (%), and cubic fraction (%), respectively, $I_m$ represents the peak area intensity near $2\theta = 28.2°$, where the peak top of the main peak of the monoclinic system appears in XRD measurement, It represents the peak area intensity near $2\theta = 30.2°$, where the peak top of the main peak of the tetragonal system appears in XRD measurement, $I_c$ represents the peak area intensity near $2\theta = 30.1°$, where the peak top of the main peak of the cubic system appears in XRD measurement, and $I_y$ represents the peak area intensity near $2\theta = 29.2°$, where the peak top of the main peak of undissolved yttria appears in XRD measurement.

Examples of preferred embodiments are described below for each of the embodiments (A-1), (A-2), (A-3), (A-4), (A-5), and (A-6).

The tetragonal content, cubic content, and monoclinic content in embodiments (A-1), (A-2), (A-3), (A-4), (A-5), and (A-6) are calculated from the formulae (2-1), (2-2), and (2-3) above, respectively. The method of measurement of tetragonal content, cubic content, and monoclinic content is as described in the EXAMPLES section below.

<Embodiment (A-1)>

A certain preferred embodiment (A-1) is, for example, a zirconia pre-sintered body in which the zirconia comprises monoclinic zirconia and tetragonal zirconia, and in which the monoclinic content is 55% or more, the tetragonal content is 10% or more, and a part of the yttria is not dissolved in zirconia as a solid solution (hereinafter, also referred to as "embodiment (A-1)").

In embodiment (A-1), the monoclinic content is preferably 55% or more, more preferably 56% or more, even more preferably 58% or more, particularly preferably 60% or more.

The monoclinic content is preferably 85% or less, more preferably 80% or less, even more preferably 75% or less, particularly preferably 70% or less.

The monoclinic content may fall in any combinations of these ranges. For example, the monoclinic content is preferably 55% or more and 85% or less, more preferably 56% or more and 80% or less, even more preferably 58% or more and 75% or less, particularly preferably 60% or more and 70% or less.

With the monoclinic content falling in these ranges, the monoclinic zirconia, when combined with a predetermined amount of tetragonal zirconia, allows the concentration difference of yttrium elements or yttrium ions to fall in the desired range within the system being sintered, and, through the combined action with the raw material powder having a predetermined average particle diameter, the resulting zirconia sintered body can exhibit superior translucency as a result of sufficient movement of the constituent metal elements or ions of the stabilizer, even with a short sintering period of 10 minutes or less.

In this specification, the concentration difference is not particularly limited, provided that the desired standard deviation range is attained in the zirconia sintered body after sintering, and the translucency satisfies formula (1).

The method of measurement of monoclinic content is as described in the EXAMPLES section below.

In embodiment (A-1), the tetragonal content is preferably 10% or more, more preferably 15% or more, even more preferably 20% or more, particularly preferably 25% or more.

The tetragonal content is preferably 44% or less, more preferably 42% or less, even more preferably 40% or less, particularly preferably 38% or less.

The tetragonal content may fall in any combinations of these ranges. For example, the tetragonal content is preferably 10% or more and 44% or less, more preferably 15% or more and 42% or less, even more preferably 20% or more and 40% or less, particularly preferably 25% or more and 38% or less.

With the tetragonal content falling in these ranges, the tetragonal zirconia, when combined with the monoclinic zirconia provided as the destination for the movement of yttrium elements or yttrium ions, allows the concentration difference of the constituent metal elements or ions of the stabilizer to fall in the desired range within the system being sintered, and, through the combined action with the raw material powder having a predetermined average particle diameter, the resulting zirconia sintered body can exhibit superior translucency as a result of sufficient movement of the constituent metal elements or ions of the stabilizer, even with a short sintering period of 10 minutes or less.

The method of measurement of tetragonal content is as described in the EXAMPLES section below.

Another certain preferred embodiment is, for example, a zirconia pre-sintered body in which the monoclinic content is 55% or more and 75% or less, and the tetragonal content is 25% or more and 45% or less.

In embodiment (A-1), yet another certain preferred embodiment is, for example, a zirconia pre-sintered body in which the proportion of the yttria not dissolved in zirconia as a solid solution is 1 to 25%.

In zirconia pre-sintered bodies according to embodiment (A-1), the proportion of yttria not dissolved in zirconia as a solid solution (hereinafter, also referred to as "undissolved yttria fraction $f_y$" or simply "$f_y$") can be calculated according to the following formula (2-4).

$$f_y \ (\%) = I_y / (I_m + I_t + I_c + I_y) \times 100 \qquad (2\text{-}4),$$

wherein $f_y$ represents the fraction of undissolved yttria (%), $I_m$ represents the peak area intensity near $2\theta = 28.2°$, where the peak top of the main peak of the monoclinic system appears in XRD measurement, It represents the peak area intensity near $2\theta = 30.2°$, where the peak top of the main peak of the tetragonal system appears in XRD measurement, $I_c$ represents the peak area intensity near $2\theta = 30.1°$, where the peak top of the main peak of the cubic system appears in XRD measurement, and $I_y$ represents the peak area intensity near $2\theta = 29.2°$, where the peak top of the main peak of undissolved yttria appears in XRD measurement.

In embodiment (A-1), in view of allowing the concentration gradient of yttrium elements to fall in the desired range with the tetragonal zirconia and monoclinic zirconia combined together, and more easily facilitating the movement of yttrium elements even with a short sintering period and yielding a zirconia sintered body with superior translucency, it is preferable that the undissolved yttria fraction $f_y$ be 1% or more, more preferably 2% or more, even more preferably 3% or more, particularly preferably 3.5% or more.

In view of reducing a decrease in the strength of the sintered body, the undissolved yttria fraction $f_y$ is preferably 25% or less. In view of reducing the standard deviation of the yttrium element distribution, and more easily facilitating the movement of yttrium elements with a short sintering time and yielding a zirconia sintered body with even superior translucency, the undissolved yttria fraction $f_y$ is more preferably 20% or less, even more preferably 18% or less, particularly preferably 16% or less.

The undissolved yttria fraction $f_y$ may fall in any combinations of these ranges. For example, the undissolved yttria fraction $f_y$ is preferably 1 to 25%, more preferably 2 to 20%, even more preferably 3 to 18%, particularly preferably 3.5 to 16%.

The undissolved yttria fraction $f_y$ can be adjusted according to factors such as the predetermined average particle diameter of the yttria powder used as a yttrium source, the content of the predetermined yttria powder in the raw material composition, and the mixing ratio.

In embodiment (A-1), the zirconia in the zirconia pre-sintered body may comprise a cubic crystal system. However, in view of more easily facilitating the movement of yttrium elements or yttrium ions, it is preferable that the zirconia pre-sintered body does not comprise a cubic crystal system. In other words, in embodiment (A-1), it is preferable that the zirconia pre-sintered body does not comprise both cubic and tetragonal crystal systems simultaneously.

<Embodiment (A-2)>

A certain preferred embodiment (A-2) is, for example, a zirconia pre-sintered body in which the zirconia comprises tetragonal zirconia, and, without corresponding to (A-1), the tetragonal content is 10% or more, and a part of the yttria is not dissolved in zirconia as a solid solution (hereinafter, also referred to as "embodiment (A-2)").

In embodiment (A-2), the zirconia in the zirconia pre-sintered body comprises a tetragonal crystal system. The tetragonal content is preferably 10% or more, more preferably 15% or more, even more preferably 20% or more, particularly preferably 25% or more.

In view of reducing a decrease in the strength of the sintered body, the tetragonal content is preferably less than 98%, more preferably 97.5% or less, even more preferably 97% or less, particularly preferably 96.5% or less.

The tetragonal content may fall in any combinations of these ranges. For example, the tetragonal content is preferably 10% or more and 98% or less, more preferably 15% or more and 97.5% or less, even more preferably 20% or more and 97% or less, particularly preferably 25% or more and 96.5% or less.

In another embodiment, it is preferable that the zirconia pre-sintered body does not comprise monoclinic zirconia, and, for example, the tetragonal content is preferably more than 45% and less than 98%, more preferably 55% or more and 97.5% or less, even more preferably 70% or more and 97% or less, particularly preferably 80% or more and 96.5% or less.

With the tetragonal content falling in these ranges, the tetragonal zirconia, when combined with undissolved yttria, allows the concentration difference of yttrium elements or yttrium ions to fall in the desired range within the system being sintered, and, through the combined action with the raw material powder having a predetermined average particle diameter, the resulting zirconia sintered body can exhibit superior translucency as a result of sufficient movement of the constituent metal elements or ions of the stabilizer, even with a short sintering period of 10 minutes or less.

The method of measurement of tetragonal content is as described in the EXAMPLES section below.

Embodiment (A-2) does not correspond to (A-1), and the monoclinic content is 0% or more and less than 55%.

The monoclinic content is preferably 1% or more, more preferably 2% or more, even more preferably 2.5% or more, particularly preferably 10% or more.

The monoclinic content is preferably less than 55%, more preferably 50% or less, even more preferably 40% or less, particularly preferably 30% or less.

The monoclinic content may fall in any combinations of these ranges. For example, the monoclinic content is preferably 1% or more and less than 55%, more preferably 2% or more and 50% or less, even more preferably 2.5% or more and 40% or less, particularly preferably 10% or more and 30% or less.

Another certain preferred embodiment is, for example, a zirconia pre-sintered body in which the zirconia in the zirconia pre-sintered body comprises a tetragonal crystal system, and in which the monoclinic content is 0%, the tetragonal content is 80% or more and 97% or less, and a part of the stabilizer is not dissolved in zirconia as a solid solution.

In embodiment (A-2), a part of the stabilizer in the yttria contained in the zirconia pre-sintered body is undissolved yttria.

In zirconia pre-sintered bodies according to embodiment (A-2), the undissolved yttria fraction $f_y$ can be calculated according to the formula (2-4) above.

In embodiment (A-2), in view of allowing the concentration gradient of yttrium elements to fall in the desired range with the tetragonal zirconia, and more easily facilitating the movement of yttrium elements even with a short sintering period and yielding a zirconia sintered body with superior translucency, it is preferable that the undissolved yttria fraction $f_y$ be more than 2%, more preferably 2.5% or more, even more preferably 3% or more, particularly preferably 3.5% or more.

In view of reducing a decrease in the strength of the sintered body, the undissolved yttria fraction $f_y$ is preferably 25% or less. In view of reducing the standard deviation of the yttrium element distribution, and more easily facilitating the movement of yttrium elements with a short sintering time and yielding a zirconia sintered body with even superior translucency, the undissolved yttria fraction $f_y$ is more preferably 20% or less, even more preferably 18% or less, particularly preferably 16% or less.

The undissolved yttria fraction $f_y$ may fall in any combinations of these ranges. For example, the undissolved yttria fraction $f_y$ is preferably 1 to 25%, more preferably 2 to 20%, even more preferably 3 to 18%, particularly preferably 3.5 to 16%.

In embodiment (A-2), the zirconia in the zirconia pre-sintered body may comprise a cubic crystal system. However, in view of more easily facilitating the movement of yttrium elements or yttrium ions, it is preferable that the zirconia pre-sintered body does not comprise a cubic crystal system. In other words, in embodiment (A-2), it is preferable that the zirconia pre-sintered body does not comprise both cubic and tetragonal crystal systems simultaneously.

<Embodiment (A-3)>

A certain preferred embodiment (A-3) is, for example, a zirconia pre-sintered body in which the zirconia comprises monoclinic zirconia and cubic zirconia, and in which the monoclinic content is 55% or more, and the cubic content is 15% or more (hereinafter, also referred to as "embodiment (A-3)").

In embodiment (A-3), the monoclinic content is preferably 55% or more, more preferably 56% or more, even more preferably 58% or more, particularly preferably 60% or more.

The monoclinic content is preferably 85% or less, more preferably 80% or less, even more preferably 75% or less, particularly preferably 70% or less.

The monoclinic content may fall in any combinations of these ranges. For example, the monoclinic content is preferably 55% or more and 85% or less, more preferably 56% or more and 80% or less, even more preferably 58% or more and 75% or less, particularly preferably 60% or more and 70% or less.

With the monoclinic content falling in these ranges, the monoclinic zirconia, when combined with a predetermined amount of cubic zirconia, and optionally undissolved yttria, allows the concentration difference of yttrium elements or yttrium ions to fall in the desired range within the system being sintered, and, through the combined action with the raw material powder having a predetermined average particle diameter, the resulting zirconia sintered body can exhibit superior translucency as a result of sufficient movement of the constituent metal elements or ions of the stabilizer, even with a short sintering period of 10 minutes or less.

The method of measurement of monoclinic content is as described in the EXAMPLES section below.

In embodiment (A-3), the cubic content is preferably 15% or more, more preferably 18% or more, even more preferably 20% or more, particularly preferably 25% or more.

The cubic content is preferably 44% or less, more preferably 42% or less, even more preferably 40% or less, particularly preferably 38% or less.

The cubic content may fall in any combinations of these ranges. For example, the cubic content is preferably 15% or more and 44% or less, more preferably 15% or more and 42% or less, even more preferably 20% or more and 40% or less, particularly preferably 25% or more and 38% or less.

With the cubic content falling in these ranges, the cubic zirconia, when combined with the monoclinic zirconia existing as the destination for the movement of yttrium elements or yttrium ions, allows the concentration difference of the constituent metal elements or ions of the stabilizer to fall in the desired range within the system being sintered, and, through the combined action with the raw material powder having a predetermined average particle diameter, the resulting zirconia sintered body can exhibit superior translucency as a result of sufficient movement of the constituent metal elements or ions of the stabilizer, even with a short sintering period of 10 minutes or less.

The method of measurement of cubic content is as described in the EXAMPLES section below.

In embodiment (A-3), yet another certain preferred embodiment is, for example, a zirconia pre-sintered body in which a part of yttria is not dissolved in zirconia as a solid solution, and the proportion of the yttria not dissolved in zirconia as a solid solution is 1 to 15%.

In zirconia pre-sintered bodies according to embodiment (A-3), the undissolved yttria fraction $f_y$ can be calculated according to the formula (2-4) above.

In embodiment (A-3), in view of allowing the concentration gradient of yttrium elements to fall in the desired range with the cubic zirconia and monoclinic zirconia combined together, and more easily facilitating the movement of yttrium elements even with a short sintering period and yielding a zirconia sintered body with superior translucency, it is preferable that the undissolved yttria fraction $f_y$ be 1% or more, more preferably 2% or more, even more preferably 3% or more, particularly preferably 3.5% or more.

In view of reducing a decrease in the strength of the sintered body, the undissolved yttria fraction $f_y$ is preferably 15% or less. In view of reducing the standard deviation of the yttrium element distribution, and more easily facilitating the movement of yttrium elements with a short sintering time and yielding a zirconia sintered body with even superior translucency, the undissolved yttria fraction $f_y$ is more preferably 14% or less, even more preferably 12% or less, particularly preferably 11% or less.

The undissolved yttria fraction $f_y$ may fall in any combinations of these ranges. For example, the undissolved yttria fraction $f_y$ is preferably 1 to 15%, more preferably 2 to 14%, even more preferably 3 to 12%, particularly preferably 3.5 to 11%.

In embodiment (A-3), the zirconia in the zirconia pre-sintered body may comprise a tetragonal crystal system. However, in view of more easily facilitating the movement of yttrium elements or yttrium ions, it is preferable that the zirconia pre-sintered body does not comprise a tetragonal crystal system. In other words, in embodiment (A-3), it is preferable that the zirconia pre-sintered body does not comprise both cubic and tetragonal crystal systems simultaneously.

<Embodiment (A-4)>

A certain preferred embodiment (A-4) is, for example, a zirconia pre-sintered body in which the zirconia comprises cubic zirconia and monoclinic zirconia, and, without corresponding to (A-3), the cubic content is 15% or more (hereinafter, also referred to as "embodiment (A-4)").

In embodiment (A-4), the zirconia in the zirconia pre-sintered body comprises cubic zirconia. The cubic content is preferably 15% or more, more preferably 20% or more, even more preferably 30% or more, particularly preferably 40% or more.

The cubic content is preferably 98% or less, more preferably 97% or less, even more preferably 96.5% or less, particularly preferably 96% or less.

The cubic content may fall in any combinations of these ranges. For example, the cubic content is preferably 15% or more and 98% or less, more preferably 20% or more and 97% or less, even more preferably 30% or more and 96.5% or less, particularly preferably 40% or more and 96% or less.

With the cubic content falling in these ranges, the cubic zirconia, when combined with the monoclinic zirconia provided as the destination for the movement of yttrium elements or yttrium ions, allows the concentration difference of the constituent metal elements or ions of the stabilizer to fall in the desired range within the system being sintered, and, through the combined action with the raw material powder having a predetermined average particle diameter, the resulting zirconia sintered body can exhibit superior translucency as a result of sufficient movement of the constituent metal elements or ions of the stabilizer, even with a short sintering period of 10 minutes or less.

The method of measurement of cubic content is as described in the EXAMPLES section below.

In embodiment (A-4), the zirconia in the zirconia pre-sintered body comprises monoclinic zirconia. The monoclinic content is preferably 1% or more, more preferably 2% or more, even more preferably 2.5% or more, particularly preferably 3% or more.

The monoclinic content is preferably less than 55%, more preferably 50% or less, even more preferably 45% or less, particularly preferably 40% or less.

The monoclinic content may fall in any combinations of these ranges. For example, the monoclinic content is preferably 1% or more and less than 55%, more preferably 2% or more and 50% or less, even more preferably 2.5% or more and 45% or less, particularly preferably 3% or more and 40% or less.

With the monoclinic content falling in these ranges, the monoclinic zirconia, when combined with the cubic zirconia, which provides yttrium elements or yttrium ions, and optionally undissolved yttria, allows the concentration difference of the constituent metal elements or ions of the stabilizer to fall in the desired range within the system being sintered, and, through the combined action with the raw material powder having a predetermined average particle diameter, the resulting zirconia sintered body can exhibit superior translucency as a result of sufficient movement of the constituent metal elements or ions of the stabilizer, even with a short sintering period of 10 minutes or less.

The method of measurement of monoclinic content is as described in the EXAMPLES section below.

In embodiment (A-4), another certain preferred embodiment is, for example, a zirconia pre-sintered body in which the zirconia comprises cubic zirconia and monoclinic zirconia, and in which the cubic content is 40% or more and 90% or less, and the monoclinic content is 10% or more and less than 55%.

In embodiment (A-4), yet another certain preferred embodiment is, for example, a zirconia pre-sintered body in which the zirconia comprises cubic zirconia and monoclinic zirconia, and in which the cubic content is 70% or more and 97% or less, and the monoclinic content is 3% or more and 30% or less.

In embodiment (A-4), still another certain preferred embodiment is, for example, a zirconia pre-sintered body in which a part of yttria is not dissolved in zirconia as a solid solution, and the proportion of the yttria not dissolved in zirconia as a solid solution is 1 to 15%.

In zirconia pre-sintered bodies according to embodiment (A-4), the undissolved yttria fraction $f_y$ can be calculated according to the formula (2-4) above.

In embodiment (A-4), in view of allowing the concentration gradient of yttrium elements to fall in the desired range with the cubic zirconia and monoclinic zirconia combined together, and more easily facilitating the movement of yttrium elements even with a short sintering period and yielding a zirconia sintered body with superior translucency, it is preferable that the undissolved yttria fraction $f_y$ be 1% or more, more preferably 2% or more, even more preferably 3% or more, particularly preferably 3.5% or more.

In view of reducing a decrease in the strength of the sintered body, the undissolved yttria fraction $f_y$ is preferably 15% or less. In view of reducing the standard deviation of the yttrium element distribution, and more easily facilitating the movement of yttrium elements with a short sintering time and yielding a zirconia sintered body with even superior translucency, the undissolved yttria fraction $f_y$ is more preferably 14% or less, even more preferably 12% or less, particularly preferably 11% or less.

The undissolved yttria fraction $f_y$ may fall in any combinations of these ranges. For example, the undissolved yttria fraction $f_y$ is preferably 1 to 15%, more preferably 2 to 14%, even more preferably 3 to 12%, particularly preferably 3.5 to 11%.

In embodiment (A-4), the zirconia in the zirconia pre-sintered body may comprise a tetragonal crystal system. However, in view of more easily facilitating the movement of yttrium elements or yttrium ions, it is preferable that the zirconia pre-sintered body does not comprise a tetragonal crystal system. In other words, in embodiment (A-4), it is preferable that the zirconia pre-sintered body does not comprise both cubic and tetragonal crystal systems simultaneously.

<Embodiment (A-5)>

A certain preferred embodiment (A-5) is, for example, a zirconia pre-sintered body in which the zirconia comprises tetragonal zirconia and cubic zirconia, and in which the tetragonal content is 5% or more and less than 50%, the cubic content is 50% or more and less than 95%, and a part of the yttria is not dissolved in zirconia as a solid solution (hereinafter, also referred to as "embodiment (A-5)").

In embodiment (A-5), the tetragonal content is preferably 6% or more, more preferably 8% or more, even more preferably 9% or more, particularly preferably 10% or more.

The tetragonal content is preferably less than 49%, more preferably less than 48%, even more preferably less than 46%, particularly preferably less than 40%.

The tetragonal content may fall in any combinations of these ranges.

For example, in certain embodiments, the tetragonal content is preferably 6% or more and less than 49%, more preferably 8% or more and less than 48%, even more preferably 9% or more and less than 46%, particularly preferably 10% or more and less than 40%.

With the tetragonal content falling in these ranges, the tetragonal zirconia, when combined with a predetermined amount of cubic zirconia along with undissolved yttria, allows the concentration difference of yttrium elements or yttrium ions to fall in the desired range within the system being sintered, and, through the combined action with the raw material powder having a predetermined average particle diameter, the resulting zirconia sintered body can exhibit superior translucency as a result of sufficient movement of the constituent metal elements or ions of the stabilizer, even with a short sintering period of 10 minutes or less.

The method of measurement of tetragonal content is as described in the EXAMPLES section below.

In embodiment (A-5), the cubic content is preferably 51% or more, more preferably 52% or more, even more preferably 54% or more, particularly preferably 60% or more.

The cubic content is preferably less than 94%, more preferably less than 92%, even more preferably less than 91%, particularly preferably less than 90%.

The cubic content may fall in any combinations of these ranges. For example, the cubic content is preferably 51% or more and less than 94%, more preferably 52% or more and less than 92%, even more preferably 54% or more and less than 91%, particularly preferably 60% or more and less than 90%.

With the cubic content falling in these ranges, the cubic zirconia, when combined with undissolved yttria along with the tetragonal zirconia existing as the destination for the movement of yttrium elements or yttrium ions, allows the concentration difference of the constituent metal elements or ions of the stabilizer to fall in the desired range within the system being sintered, and, through the combined action with the raw material powder having a predetermined average particle diameter, the resulting zirconia sintered body can exhibit superior translucency as a result of sufficient movement of the constituent metal elements or ions of the stabilizer, even with a short sintering period of 10 minutes or less.

The method of measurement of cubic content is as described in the EXAMPLES section below.

In embodiment (A-5), a part of the stabilizer in the yttria contained in the zirconia pre-sintered body is undissolved yttria.

In zirconia pre-sintered bodies according to embodiment (A-5), the undissolved yttria fraction $f_y$ can be calculated according to the formula (2-4) above.

In embodiment (A-5), in view of allowing the concentration gradient of yttrium elements to fall in the desired range with the cubic zirconia and tetragonal zirconia combined together, and more easily facilitating the movement of yttrium elements even with a short sintering period and yielding a zirconia sintered body with superior translucency, it is preferable that the undissolved yttria fraction $f_y$ be 0.001% or more, more preferably 0.01% or more, even more preferably 0.02% or more, particularly preferably 0.05% or more, most preferably 0.1% or more.

In view of reducing a decrease in the strength of the sintered body, the undissolved yttria fraction $f_y$ is preferably 12% or less. In view of reducing the standard deviation of the yttrium element distribution, and more easily facilitating the movement of yttrium elements with a short sintering time and yielding a zirconia sintered body with even superior translucency, the undissolved yttria fraction $f_y$ is more preferably 10% or less, even more preferably 9% or less, particularly preferably 8% or less, most preferably 5% or less.

The undissolved yttria fraction $f_y$ may fall in any combinations of these ranges. For example, the undissolved yttria fraction $f_y$ is preferably 0.001 to 12%, more preferably 0.01 to 10%, even more preferably 0.02 to 9%, particularly preferably 0.05 to 8%, most preferably 0.1 to 5%.

In embodiment (A-5), in view of more easily facilitating the movement of yttrium elements or yttrium ions, it is preferable that the zirconia in the zirconia pre-sintered body does not comprise a monoclinic crystal system.

<Embodiment (A-6)>

A certain preferred embodiment (A-6) is, for example, a zirconia pre-sintered body in which the zirconia comprises tetragonal zirconia and cubic zirconia, and, without corresponding to (A-5), the cubic content is 10% or more and less than 50%, and a part of the yttria is not dissolved in zirconia as a solid solution (hereinafter, also referred to as "embodiment (A-6)").

Embodiment (A-6) does not correspond to (A-5), and the tetragonal content is 50% or more and less than 90%.

In embodiment (A-6), the tetragonal content is preferably 51% or more, more preferably 52% or more, even more preferably 54% or more, particularly preferably 60% or more.

The tetragonal content is preferably less than 89%, more preferably less than 88%, even more preferably less than 86%, particularly preferably less than 85%.

The tetragonal content may fall in any combinations of these numerical ranges.

For example, in certain embodiments, the tetragonal content is preferably 51% or more and less than 89%, more preferably 52% or more and less than 88%, even more preferably 54% or more and less than 86%, particularly preferably 60% or more and less than 85%.

With the tetragonal content falling in these ranges, the tetragonal zirconia, when combined with a predetermined amount of cubic zirconia along with undissolved yttria, allows the concentration difference of yttrium elements or yttrium ions to fall in the desired range within the system being sintered, and, through the combined action with the raw material powder having a predetermined average particle diameter, the resulting zirconia sintered body can exhibit superior translucency as a result of sufficient movement of the constituent metal elements or ions of the stabilizer, even with a short sintering period of 10 minutes or less.

The method of measurement of tetragonal content is as described in the EXAMPLES section below.

In embodiment (A-6), the cubic content is preferably 11% or more, more preferably 12% or more, even more preferably 14% or more, particularly preferably 15% or more.

The cubic content is preferably less than 49%, more preferably less than 48%, even more preferably less than 46%, particularly preferably less than 40%.

The cubic content may fall in any combinations of these ranges. For example, the cubic content is preferably 11% or more and less than 49%, more preferably 12% or more and less than 48%, even more preferably 14% or more and less than 46%, particularly preferably 15% or more and less than 40%.

With the cubic content falling in these ranges, the cubic zirconia, when combined with undissolved yttria along with the tetragonal zirconia existing as the destination for the movement of yttrium elements or yttrium ions, allows the concentration difference of the constituent metal elements or ions of the stabilizer to fall in the desired range within the system being sintered, and, through the combined action with the raw material powder having a predetermined average particle diameter, the resulting zirconia sintered body can exhibit superior translucency as a result of sufficient movement of the constituent metal elements or ions of the stabilizer, even with a short sintering period of 10 minutes or less.

The method of measurement of cubic content is as described in the EXAMPLES section below.

In embodiment (A-6), a part of the stabilizer in the yttria contained in the zirconia pre-sintered body is undissolved yttria.

In zirconia pre-sintered bodies according to embodiment (A-6), the undissolved yttria fraction $f_y$ can be calculated according to the formula (2-4) above.

In embodiment (A-6), in view of allowing the concentration gradient of yttrium elements to fall in the desired range with the cubic zirconia and tetragonal zirconia combined together, and more easily facilitating the movement of yttrium elements even with a short sintering period and yielding a zirconia sintered body with superior translucency, it is preferable that the undissolved yttria fraction $f_y$ be 0.001% or more, more preferably 0.01% or more, even more preferably 0.02% or more, particularly preferably 0.05% or more, most preferably 0.1% or more.

In view of reducing a decrease in the strength of the sintered body, the undissolved yttria fraction $f_y$ is preferably 12% or less. In view of reducing the standard deviation of the yttrium element distribution, and more easily facilitating the movement of yttrium elements with a short sintering time and yielding a zirconia sintered body with even superior translucency, the undissolved yttria fraction $f_y$ is more preferably 10% or less, even more preferably 9% or less, particularly preferably 8% or less, most preferably 5% or less.

The undissolved yttria fraction $f_y$ may fall in any combinations of these ranges. For example, the undissolved yttria fraction $f_y$ is preferably 0.001 to 12%, more preferably 0.01 to 10%, even more preferably 0.02 to 9%, particularly preferably 0.05 to 8%, most preferably 0.1 to 5%.

In embodiment (A-6), in view of more easily facilitating the movement of yttrium elements or yttrium ions, it is preferable that the zirconia in the zirconia pre-sintered body does not comprise a monoclinic crystal system.

In embodiments (A-1) to (A-6), the stabilizer is yttria, and the standard deviation of the yttrium element distribution falls in a range of 2 mol% or more and less than 21 mol%. This allows the movement of yttrium elements or yttrium ions to sufficiently proceed between materials within the system being sintered, even with a short sintering period of 10 minutes or less, yielding a zirconia sintered body satisfying the formula (1) regarding its translucency.

As described above, in view of ease of attaining the desired range for the standard deviation of the yttrium element distribution, it is preferable that a zirconia pre-sintered body of the present invention excludes the following, for example.

a zirconia pre-sintered body in which the zirconia consists of tetragonal zirconia and cubic zirconia, and that does not comprise a stabilizer not dissolved in zirconia as a solid solution;

a zirconia pre-sintered body in which the zirconia consists of cubic zirconia, and a part of the stabilizer is not dissolved in zirconia as a solid solution;

a zirconia pre-sintered body in which the zirconia consists of cubic zirconia, and that does not comprise a stabilizer not dissolved in zirconia as a solid solution;

a zirconia pre-sintered body in which the zirconia consists of monoclinic zirconia and tetragonal zirconia, and that does not comprise a stabilizer not dissolved in zirconia as a solid solution; and

a zirconia pre-sintered body in which the zirconia consists of tetragonal zirconia, and that does not comprise a stabilizer not dissolved in zirconia as a solid solution.

Certain embodiments may exclude zirconia pre-sintered bodies with a porosity of 15 to 30%. The porosity is a calculated value according to the following formula.

$$\text{Porosity (\%)} = \text{pore volume} / (\text{pore volume} + \text{skeletal volume}) \times 100$$

The pore volume is a value determined through the mercury intrusion method by measuring interconnected pores that do not include closed pores with a diameter of about 5 nm to 250 $\mu$m. The skeletal volume is a calculated value obtained from the true density measured through the gas displacement method. The skeletal volume is calculated as skeletal volume ($cm^3$/g) = 1/true density (g/$cm^3$).

The pore volume and skeletal volume can be determined using a zirconia pre-sintered body machined into a prism-shaped sample (5 mm $\times$ 5 mm $\times$ 5 mm). The pore volume can be measured using an automated multifunctional mercury porosimeter (PoreMaster, manufactured by Anton Paar, Japan) (measurement conditions include mercury surface tension: 480 erg/$cm^2$, contact angle: 140°, discharge contact angle: 140°, pressure: 0 to 50,000 psia). For the measurement of skeletal volume, it can be calculated by measuring the true density with a dry automatic densitometer (AccuPyc II 1340, Shimadzu Corporation).

A zirconia pre-sintered body of the present invention has a density of preferably 3.6 g/$cm^3$ or less, more preferably 3.5 g/$cm^3$ or less, even more preferably 3.4 g/$cm^3$ or less.

A zirconia pre-sintered body of the present invention has a density of preferably 2.5 g/$cm^3$ or more, more preferably 2.7 g/$cm^3$ or more, even more preferably 2.9 g/$cm^3$ or more.

The density of the zirconia pre-sintered body can be calculated as the mass-to-volume ratio (mass of zirconia pre-sintered body) / (volume of zirconia pre-sintered body). For example, the density of the zirconia pre-sintered body can be determined by cutting out test specimens with dimensions of 10 mm $\times$ 10 mm $\times$ 10 mm from arbitrary locations of the zirconia pre-sintered body (n = 3), measuring and mass and volume of each test specimen, and averaging the measured values. The calculated average can then be applied to the above formula to determine the density.

A zirconia pre-sintered body of the present invention has an average primary particle diameter of preferably 40 nm or more, more preferably 45 nm or more, even more preferably 50 nm or more.

A zirconia pre-sintered body of the present invention has an average primary particle diameter of preferably 110 nm or less, more preferably 105 nm or less, even more preferably 100 nm or less.

The average primary particle diameter of the zirconia pre-sintered body may fall in any combinations of these ranges. For example, the average primary particle diameter is preferably 40 to 110 nm, more preferably 45 to 105 nm, even more preferably 50 to 100 nm.

The measurement method for the average primary particle diameter of the zirconia pre-sintered body is as described in the EXAMPLES section below.

A zirconia pre-sintered body of the present invention may comprise additives other than zirconia and stabilizers, provided that the present invention can exhibit its effects. Examples of such additives include colorants (including pigments, composite pigments, and fluorescent agents), binders, dispersants, emulsifiers, antifoaming agents, pH adjusters, lubricants, and translucency adjusters. The additives may be used alone, or two or more thereof may be used in combination.

Examples of the pigments include oxides of at least one element selected from the group consisting of Ti, V, Cr, Mn, Fe, Co, Ni, Zn, Y, Zr, Sn, Sb, Bi, Ce, Sm, Eu, Gd, and Er.

Examples of the composite pigments include $(Zr,V)O_2$, $Fe(Fe,Cr)_2O_4$, $(Ni,Co,Fe)(Fe,Cr)_2O_4 \cdot ZrSiO_4$, and $(Co,Zn)Al_2O_4$.

A zirconia pre-sintered body of the present invention may comprise a fluorescent agent. The zirconia sintered body can exhibit fluorescence by incorporating a fluorescent agent in the zirconia pre-sintered body. The type of fluorescent agent is not particularly limited, and it is possible to use a single fluorescent agent or two or more fluorescent agents capable of emitting fluorescence at any wavelength of light.

Examples of the fluorescent agent include those containing metal elements. Examples of the metal elements include Ga, Bi, Ce, Nd, Sm, Eu, Gd, Tb, Dy, and Tm. The fluorescent agent may contain one of these metal elements by itself, or may contain two or more of these metal elements. Preferred among these metal elements are Ga, Bi, Eu, Gd, and Tm, more preferably Bi and Eu.

Examples of the fluorescent agent include oxides, hydroxides, acetates, and nitrates of the metal elements above. The fluorescent agent may be, for example, $Y_2SiO_5$:Ce, $Y_2SiO_5$:Tb, $(Y,Gd,Eu)BO_3$, $Y_2O_3$:Eu, YAG:Ce, $ZnGa_2O_4$:Zn, or $BaMgAl_{10}O_{17}$:Eu.

The content of the fluorescent agent in the zirconia pre-sintered body is not particularly limited, and may be appropriately adjusted according to factors such as the type of fluorescent agent, and the intended use of the zirconia sintered body. However, in view of considerations such as suitability as dental prostheses, the content of fluorescent agent is preferably 0.001 mass% or more, more preferably 0.005 mass% or more, even more preferably 0.01 mass% or more in terms of an oxide of metal elements contained in the fluorescent agent, relative to 100 mass% of zirconia within the zirconia pre-sintered body.

The content of fluorescent agent is preferably 1 mass% or less, more preferably 0.5 mass% or less, even more preferably 0.1 mass% or less in terms of an oxide of metal elements contained in the fluorescent agent. When the content is at or above these lower limits, the fluorescence can match or exceed that of natural human teeth. When the content is at or below the foregoing upper limits, it is possible to reduce a decrease in translucency and mechanical strength.

Examples of the binders include polyvinyl alcohol, methyl cellulose, carboxymethyl cellulose, acrylic binders, wax binders, polyvinyl butyral, polymethyl methacrylate, and ethyl cellulose. For improved translucency, the binder content in a zirconia composition of the present invention is preferably 10 mass% or less, more preferably 5 mass% or less, even more preferably 3 mass% or less relative to 100 mass% of zirconia.

Examples of the plasticizers include polyethylene glycol, glycerin, propylene glycol, and dibutyl phthalate.

Examples of the dispersants include ammonium polycarboxylate (such as triammonium citrate), ammonium polyacrylate, acryl copolymer resins, acrylic acid ester copolymers, polyacrylic acid, bentonite, carboxymethyl cellulose, anionic surfactants (for example, polyoxyethylene alkyl ether phosphoric acid esters such as polyoxyethylene lauryl ether phosphoric acid ester), non-ionic surfactants, oleic glyceride, amine salt surfactants, oligosaccharide alcohols, and stearic acid.

Examples of the emulsifiers include alkyl ethers, phenyl ethers, sorbitan derivatives, and ammonium salts.

Examples of the antifoaming agents include alcohols, polyethers, polyethylene glycols, silicone, and waxes.

Examples of the pH adjusters include ammonia, ammonium salts (including ammonium hydroxides such as tetramethylammonium hydroxide).

Examples of the lubricants include polyoxyethylene alkylate ethers, and waxes.

Examples of the translucency adjusters include aluminum oxide ($Al_2O_3$), titanium oxide ($TiO_2$), silicon dioxide ($SiO_2$), zircon, lithium silicate, and lithium disilicate.

[Production Method of Zirconia Pre-Sintered Body]

A zirconia pre-sintered body of the present invention can be produced by firing (pre-sintering) the zirconia composition (for example, a molded body) to such an extent that zirconia particles do not fully sinter.

In this specification, the zirconia composition refers to a composition comprising a zirconia powder, and a powder of a stabilizer capable of preventing a phase transformation of zirconia.

The zirconia composition may be, for example, a molded body that has been molded.

The molded body refers to a material that has been molded by applying an external force to a powder that contains zirconia-based particles. Because the zirconia composition and zirconia molded body are in the unfired state, these refer to materials that have not formed necks (no necking).

A certain embodiment is, for example, a zirconia composition comprising zirconia, and a stabilizer capable of preventing a phase transformation of zirconia, and satisfying the formula (1) below when $\Delta L_1^*(W\text{-}B)$ related to a first sintered body fabricated by sintering the zirconia composition at 1,550°C for 120 minutes is compared to $\Delta L_2^*(W\text{-}B)$ related to a second sintered body fabricated by sintering the zirconia composition at 1,550°C for 10 minutes,

$$\Delta L_2^*(W\text{-}B) \,/\, \Delta L_1^*(W\text{-}B) \geq 0.85 \qquad (1).$$

$\Delta L_1^*(W\text{-}B)$ and $\Delta L_2^*(W\text{-}B)$ are as described in conjunction with the zirconia pre-sintered body.

The pre-sintering of the zirconia composition results in the removal of organic materials, and yields a zirconia pre-sintered body in which the desired amount of stabilizer has dissolved in zirconia as a solid solution, and the primary particles have formed necks.

In view of achieving excellent translucency in a sort time period in the subsequent sintering step, and removing organic materials and preventing adverse effects on the sintering process, the firing temperature (pre-sintering temperature) of the zirconia composition is preferably 200°C or more, more preferably 300°C or more, even more preferably 400°C or more.

The pre-sintering temperature is preferably 900°C or less, more preferably 700°C or less, even more preferably 600°C or less.

The pre-sintering temperature may fall in any combinations of these ranges. For example, the pre-sintering temperature is preferably 200 to 900°C, more preferably 300 to 700°C, even more preferably 400 to 600°C.

There is no specific restriction on the pressure during pre-sintering, and it may be ordinary pressure.

The duration of the process at the pre-sintering temperature (pre-sintering time) is preferably 30 minutes or more, more preferably 120 minutes or more. A pre-sintering time of 120 minutes or more allows for the removal of organic materials, easily preventing adverse effects on the subsequent sintering process.

The pre-sintering time is preferably 360 minutes or less, more preferably 240 minutes or less. A pre-sintering time of 240 minutes or less is preferred from the standpoint of limiting the diffusion distance of the stabilizer and maintaining its concentration gradient, and achieving excellent translucency in a short time period in the subsequent sintering process.

The pre-sintering time may fall in any combinations of these ranges. For example, the pre-sintering time is preferably 30 to 360 minutes, more preferably 120 to 240 minutes.

[Production Method of Zirconia Composition]

A method of production of the zirconia composition is, for example, a method that comprises a step of producing a zirconia powder, a step of producing a powder of a stabilizer capable of preventing a phase transformation of zirconia (hereinafter, also referred to as "stabilizer powder"), and a step of mixing the zirconia powder and the stabilizer powder to produce a powder containing zirconia-based particles.

In this specification, the term "zirconia-based particles" is used to refer to particles containing zirconia particles and stabilizer particles.

· Method of Production of Zirconia Powder and Stabilizer Powder

There is no particular restriction on the method of preparation of the zirconia powder and yttria powder representing raw material powders. It is possible to employ a method, for example, such as the breakdown process, in which coarse particles are pulverized or crushed into a fine powder, or the building-up process, which synthesizes particles through nucleation and growth from atoms or ions.

The zirconia powder includes, for example, a tetragonal zirconia powder (T), a monoclinic zirconia powder (M), and a cubic zirconia powder (C).

In view of yielding a zirconia pre-sintered body with excellent translucency by short sintering, the zirconia powder has an average primary particle diameter (r1) (hereinafter, also referred to as "average particle diameter (r1)") of preferably 40 nm or more, more preferably 45 nm or more, even more preferably 50 nm or more.

In view of yielding a zirconia pre-sintered body with excellent translucency by short sintering, the zirconia powder has an average particle diameter (r1) of preferably 110 nm or less, more preferably 105 nm or less, even more preferably 100 nm or less.

The average particle diameter (r1) of zirconia powder may fall in any combinations of these ranges. For example, the average particle diameter (r1) of the zirconia powder (zirconia powder (T), zirconia powder (C), or zirconia powder (M)) is preferably 40 to 110 nm, more preferably 45 to 105 nm, even more preferably 50 to 100 nm.

With the average particle diameters of the tetragonal zirconia powder (T), monoclinic zirconia powder (M), and cubic zirconia powder (C) falling in these ranges of average particle diameter (r1), the resulting zirconia sintered body can exhibit superior translucency as a result of sufficient movement of the constituent metal elements or ions of the stabilizer when a specific crystal system(s) is adopted, even with a short sintering period of 10 minutes or less.

In view of yielding a zirconia pre-sintered body with excellent translucency by short sintering, the stabilizer powder capable of preventing a phase transformation of zirconia has an average primary particle diameter (r2) (hereinafter, also referred to as "average particle diameter (r2)") of preferably 20 nm or more, more preferably 40 nm or more, even more preferably 45 nm or more, particularly preferably 50 nm or more.

In view of yielding a zirconia pre-sintered body with excellent translucency by short sintering, the stabilizer powder has an average particle diameter (r2) of preferably 350 nm or less, more preferably 300 nm or less, even more preferably 250 nm or less, particularly preferably 200 nm or less.

The average particle diameter (r2) of the stabilizer powder may fall in any combinations of these ranges. For example, the average particle diameter (r1) of the zirconia powder is preferably 20 to 350 nm, more preferably 40 to 300 nm, even more preferably 45 to 250 nm, particularly preferably 50 to 200 nm, most preferably 60 to 110 nm.

With the average particle diameter (r2) of the stabilizer powder falling in these ranges, the resulting zirconia sintered body can exhibit superior translucency as a result of sufficient movement of the constituent metal elements or ions of the stabilizer when combined with the zirconia powder of a specific crystal system and the predetermined average particle diameter (r1), even with a short sintering period of 10 minutes or less.

In certain embodiments, the stabilizer powder may have an average particle diameter (r2) of more than 60 nm. An example of such embodiments is a zirconia composition in which the stabilizer powder has an average particle diameter (r2) of more than 60 nm and 300 nm or less.

The average particle diameters (r1) and (r2) represent the average primary particle diameters. For example, the average particle diameters (r1) and (r2) can be measured with a laser diffraction/scattering particle size distribution analyzer (Partica LA-950 manufactured by Horiba Ltd. under this trade name), where a slurry diluted with water is subjected to ultrasonic waves for 30 minutes, and subsequently measured by volume under continued ultrasonic radiation.

The following describes examples of the preparation method of zirconia powder. The method of preparation of zirconia powder can also be applied to the preparation of stabilizer particles, unless specifically stated otherwise.

A certain preferred embodiment is, for example, a zirconia composition that satisfies the following condition (i) or (ii),

(i) the zirconia comprises a tetragonal zirconia powder (T), and a part of the stabilizer is a stabilizer powder that is not dissolved in zirconia as a solid solution;

(ii) the zirconia comprises a monoclinic zirconia powder (M) and a cubic zirconia powder (C).

Examples of preferred embodiments satisfying the condition (i) include:

a zirconia composition in which the zirconia consists of a tetragonal zirconia powder (T), and a part of the stabilizer is a stabilizer powder that is not dissolved in zirconia as a solid solution;
a zirconia composition in which the zirconia comprises a monoclinic zirconia powder (M) and a tetragonal zirconia powder (T), and a part of the stabilizer is a stabilizer powder that is not dissolved in zirconia as a solid solution; and
a zirconia composition in which the zirconia comprises a tetragonal zirconia powder (T) and a cubic zirconia powder (C), and a part of the stabilizer is a stabilizer powder that is not dissolved in zirconia as a solid solution.

Another certain preferred embodiment is, for example, a zirconia composition that satisfies the condition (i), and additionally comprises a monoclinic zirconia powder (M).

Yet another certain preferred embodiment is, for example, a zirconia composition that satisfies the condition (ii), and in which a part of the stabilizer is not dissolved in zirconia as a solid solution.

The zirconia raw material used for the production of zirconia powder may be tetragonal zirconia, monoclinic zirconia, or cubic zirconia. Such zirconia raw materials may be materials produced using the method described in, for example, Japanese Patent No. 6543926 (those produced by hydrolysis reaction), or may be commercially available products.

Examples of commercially available products include zirconia powder TZ-0 (monoclinic crystal 0Y (0 mol% yttria)), zirconia powder TZ-3Y-E with 3 mol% dissolved yttria (tetragonal crystal 3Y), zirconia powder TZ-6Y with 6 mol% dissolved yttria (cubic crystal 6Y), and zirconia powder TZ-10Y with 10 mol% dissolved yttria (cubic crystal 10Y), all manufactured by Tosoh Corporation.

In the breakdown process, coarse zirconia raw material particles can be individually pulverized according to their crystal systems to produce the tetragonal zirconia powder (T), monoclinic zirconia powder (M), and cubic zirconia powder (C), each adjusted to meet the foregoing range of average particle diameter (r2).

In view of ease of controlling the average particle diameters (r1) and (r2) to fall within the desired ranges, it is preferable to prepare the zirconia powder by pulverizing the zirconia raw material, separately from the raw material of the stabilizer powder (for example, yttria raw material).

Similarly, the raw material compound (for example, yttria) for the stabilizer powder can be pulverized using a known method (for example, ball milling) to adjust it to the average particle diameter (r2). The average particle diameter of the raw material compound is not particularly limited, as long as it can be adjusted to fall within the average particle diameter (r2) range through pulverization.

Regarding the zirconia powder with the average particle diameter (r1) and the stabilizer powder with the average particle diameter (r2), the average particle diameter can be adjusted using known methods, such as pulverizing the raw material powder.

In view of ease of adjustment to the predetermined average particle diameter (r1) and average particle diameter (r2), it is preferable to use a micro-sized pulverization media for pulverization, for example, pulverization media with a size of 100 μm or less.

Preferably, the zirconia powder obtained through the pulverization of coarse particles is classified.

Known methods and devices can be used for classification. As an example of known methods, the zirconia powder may be classified by elutriation, which takes advantage of differences in sedimentation speed due to dispersibility based on particle size, with the process optionally accelerated with a centrifuge. Examples of known devices include porous membranes (such as membrane filters with a pore size of 100 nm), and classifiers (such as wet classifiers and dry classifiers).

The raw material powder can have the desired average particle diameter through procedures such as pulverization and classification, including adjustments of the pulverization time as needed.

When the raw material powder has the desired average particle diameter, it is easier to confine the concentration gradient of yttrium elements or yttrium ions within the predetermined range when a predetermined crystal system and/or undissolved yttria are adopted in the crystal system of zirconia. Presumably, this leads to mass transfer as the particles consolidate during sintering, allowing the movement of yttrium elements or yttrium ions to sufficiently proceed even with a short sintering time of 10 minutes or less, and yielding a zirconia sintered body that has a more uniform distribution of yttrium elements, satisfying the formula (1) above regarding its translucency.

The following explanation will be given through the case where the stabilizer is yttria; however, the present invention is not restricted to cases where the stabilizer is yttria.

The content of yttria dissolved in raw material tetragonal zirconia as a solid solution (hereinafter, also referred to as "amount of dissolved yttria) is preferably 2 mol% or more, more preferably 2.2 mol% or more, even more preferably 2.5 mol% or more relative to the total mole of zirconia and yttria.

The amount of dissolved yttria in the tetragonal zirconia is preferably less than 5 mol%, more preferably 4.8 mol% or less, even more preferably 4.5 mol% or less, particularly preferably 4.0 mol% or less, most preferably 3.8 mol% or less.

The amount of dissolved yttria in the tetragonal zirconia may fall in any combinations of these ranges. For example, the amount of dissolved yttria in the tetragonal zirconia is preferably 2 mol% or more and less than 5 mol%, more preferably 2.0 mol% or more and 4.8 mol% or less, even more preferably 2.2 mol% or more and 4.5 mol% or less, particularly preferably 2.2 mol% or more and 4.0 mol% or less, most preferably 2.5 mol% or more and 3.8 mol% or less.

The amount of dissolved yttria in the zirconia raw material is also applicable to that in the tetragonal zirconia powder (T) obtained from the zirconia raw material.

The amount of dissolved yttria in the monoclinic zirconia used in present invention is preferably 0 to 1 mol%, more preferably 0 to 0.5 mol%, even more preferably 0 mol% relative to the total mole of zirconia and yttria.

The amount of dissolved yttria in the zirconia raw material is also applicable to that in the monoclinic zirconia powder (M) obtained from the zirconia raw material.

The amount of dissolved yttria in the raw material cubic zirconia is preferably 5 mol% or more, more preferably 5.5 mol% or more, even more preferably 6 mol% or more relative to the total mole of zirconia and yttria.

The amount of dissolved yttria in the cubic zirconia is preferably 15 mol% or less, more preferably 12 mol% or less, even more preferably 10 mol% or less.

The amount of dissolved yttria in the cubic zirconia may fall in any combinations of these ranges. For example, the amount of dissolved yttria in the cubic zirconia is preferably more than 5 mol% and 15 mol% or less, more preferably 5.5 mol% or more and 12 mol% or less, even more preferably 6 mol% or more and 10 mol% or less.

The amount of dissolved yttria in the zirconia raw material is also applicable to that in the cubic zirconia powder (C) obtained from the zirconia raw material.

The amount of dissolved yttria in tetragonal, monoclinic, and cubic zirconia can be measured using techniques, for example, such as inductively coupled plasma (ICP) emission spectral analysis, and X-ray fluorescence analysis (XRF).

A zirconia composition of the present invention may comprise additives other than zirconia and stabilizers, provided that the present invention can exhibit its effects. Examples of such additives include colorants (including pigments, composite pigments, and fluorescent agents), binders, dispersants, emulsifiers, antifoaming agents, pH adjusters, lubricants, alumina ($Al_2O_3$), titanium oxide ($TiO_2$), and silica ($SiO_2$). The additives may be used alone, or two or more thereof may be used in combination. The additives may be the same additives exemplified for the zirconia pre-sintered body.

The additives may be added while mixing or pulverizing the raw material, or may be added to the powder after pulverization.

In producing a zirconia composition of the present invention, the zirconia powder and the stabilizer powder may be mixed by either a dry or wet process.

The mixing ratio of zirconia powder and stabilizer powder may be appropriately adjusted to achieve the desired content of crystal system, depending on the embodiment (for example, embodiments (A-1) to (A-6)).

For example, when zirconia compositions satisfying condition (i) additionally comprise a monoclinic zirconia powder (M) as needed, the ratio of the total mass of zirconia powder (T) and zirconia powder (M) to the mass of the stabilizer powder is preferably 85.0 mass%:15.0 mass% to 99.8 mass%:0.2 mass%.

In zirconia compositions satisfying condition (i), the content of zirconia powder (T) and the content of zirconia powder (M) can be individually adjusted within the specified ranges to achieve the tetragonal content and monoclinic content specified in embodiments (A-1) and (A-2).

In certain embodiments, when zirconia compositions satisfying condition (i) comprise zirconia powder (M) as in embodiments (A-1) and (A-2), the content of the zirconia powder (M) in the zirconia compositions is preferably 0 to 85 mass%, more preferably 0 to 82 mass%, even more preferably 0 to 80 mass% in the total mass of the zirconia powder (T) and the zirconia powder (M).

Preferred as zirconia compositions satisfying condition (i) are those in which the zirconia powder (T) comprises a dissolved stabilizer capable of preventing a phase transformation of zirconia.

In zirconia powder (T), the content of the dissolved stabilizer capable of preventing a phase transformation of zirconia is preferably 2 to 4 mol%, more preferably 2 to 3.5 mol%, even more preferably 2 to 3 mol% relative to the total mole of the zirconia and stabilizer.

In zirconia compositions satisfying condition (ii), the ratio of the total mass of the zirconia powder (M) and the zirconia powder (C) to the mass of the stabilizer powder is preferably 85.0 mass%:15.0 mass% to 100 mass%:0 mass%.

In zirconia compositions satisfying condition (ii), the content of zirconia powder (M) and the content of zirconia powder (C) can be individually adjusted to fall within the specified ranges to achieve the monoclinic content and cubic content specified in embodiments (A-3) and (A-4).

In certain embodiments, the content of the zirconia powder (C) in zirconia compositions satisfying condition (ii) is preferably 15.0 to 95.0 mass%, more preferably 18.0 to 82.0 mass%, even more preferably 20.0 to 80.0 mass% in the total mass of the zirconia powder (M) and the zirconia powder (C).

In zirconia compositions satisfying condition (ii), the content of the dissolved stabilizer capable of preventing a phase transformation of zirconia in zirconia powder (M) is preferably 0 to 1 mol%, more preferably 0 to 0.5 mol%, even more preferably 0 mol% relative to the total mole of the zirconia and stabilizer.

In zirconia compositions satisfying condition (ii), the content of the dissolved stabilizer capable of preventing a phase transformation of zirconia in zirconia powder (C) is preferably 5 mol% or more and 15 mol% or less, more preferably 5.5 mol% or more and 12 mol% or less, even more preferably 6 mol% or more and 10 mol% or less relative to the total mole of the zirconia and stabilizer.

When zirconia compositions satisfying condition (i) additionally comprise a cubic zirconia powder (T) as needed, the ratio of the total mass of the zirconia powder (T) and the zirconia powder (C) to the mass of the stabilizer powder is preferably 88.0 mass%:12.0 mass% to 99.999 mass%:0.001 mass%, more preferably 90.0 mass%:10.0 mass% to 99.99 mass%:0.01 mass%, even more preferably 91.0 mass%:9.0 mass% to 99.98 mass%:0.02 mass%, particularly preferably 92.0 mass%:8.0 mass% to 99.95 mass%:0.05 mass%, most preferably 95.0 mass%:5.0 mass% to 99.9 mass%:0.1 mass%.

When zirconia compositions satisfying condition (i) additionally comprise a cubic zirconia powder (T) as needed, the content of zirconia powder (T) and the content of zirconia powder (C) can be individually adjusted to fall within the specified ranges to achieve the tetragonal content and cubic content specified in embodiments (A-5) and (A-6).

The solvent used for wet mixing is not particularly limited, as long as it contains water. The solvent may be a mixed solvent using an organic solvent with water, or may be a solvent using water alone.

Examples of organic solvents include:

> alcohols such as methanol, ethanol, 1-propanol, 2-propanol, 1-butanol, 2-butanol, 2-methyl-2-propanol, 2-methoxyethanol, 2-ethoxyethanol, 2-(2-ethoxyethoxy)ethanol, diethylene glycol monobutyl ether, and glycerin;
> ketones such as acetone, and methyl ethyl ketone;
> ethers such as tetrahydrofuran, diethyl ether, diisopropyl ether, 1,4-dioxane, and dimethoxyethane (including modified ethers (preferably, ether-modified ethers and/or ester-modified ethers, more preferably ether-modified alkylene glycols and/or ester-modified alkylene glycols), such as propylene glycol monomethyl ether acetate (commonly known as PGMEA));
> esters such as ethyl acetate, and butyl acetate;
> hydrocarbons such as hexane and toluene; and
> halogenated hydrocarbons such as chloroform, and carbon tetrachloride.

The organic solvent may be used alone, or two or more thereof may be used in combination.

A zirconia composition of the present invention may be in a dry state, or in a liquid state containing liquid or contained in liquid. For example, the zirconia composition may have a form of a powder, a paste, or a slurry.

The method for wet mixing the raw materials in a solvent containing water is not particularly limited. For example, the raw materials may be formed into a slurry containing zirconia-based particles through wet pulverization and mixing using a known pulverizing mixer (such as a ball mill). The slurry containing zirconia-based particles can then be dried and granulated to produce a granular zirconia composition.

In the following, a mixed powder resulting from the mixing of zirconia powder and stabilizer powder is also referred to as a "powder containing zirconia-based particles".

When drying and granulating the slurry, the drying method is not particularly limited, and may be, for example, atomization drying (spray drying), supercritical drying, freeze drying, hot-air drying, or vacuum drying. For considerations such as the capability to inhibit particle aggregation during drying and provide a denser zirconia sintered body, the preferred method is any one of atomization drying, supercritical drying, and freeze drying, more preferably atomization drying or supercritical drying, even more preferably atomization drying.

The zirconia-based particle-containing slurry subjected to drying may be a slurry containing water as a dispersion medium. However, for considerations such as the capability to inhibit particle aggregation during drying and provide a denser zirconia sintered body, the slurry is preferably one containing a dispersion medium other than water, such as organic solvents.

For considerations such as the capability to inhibit particle aggregation during drying and provide a denser zirconia sintered body, the water content of the zirconia-based particle-containing slurry subjected to drying is preferably 3 mass% or less, more preferably 1 mass% or less, even more preferably 0.1 mass% or less. The water content can be measured using a Karl Fischer aquameter.

The drying conditions in the foregoing drying methods are not particularly limited, and known drying conditions may be appropriately adopted. When using an organic solvent as a dispersion medium, it is preferable that drying be performed in the presence of a nonflammable gas, more preferably nitrogen, in order to reduce the risk of explosion during drying.

In the case of supercritical drying, the supercritical fluid is not particularly limited, and may be, for example, water or carbon dioxide. However, the supercritical fluid is preferably carbon dioxide for considerations such as the capability to inhibit particle aggregation and provide a denser zirconia sintered body.

When employing atomization drying in particular, it is preferable that the dispersion medium of the zirconia-based particle-containing slurry subjected to drying contain a liquid with a surface tension at 25°C of 50 mN/m or less because it enables the mitigation of zirconia particle aggregation during drying, and the production of a denser zirconia sintered body. In view of these considerations, the liquid has a surface tension of preferably 40 mN/m or less, more preferably 30 mN/m or less.

The surface tension at 25°C may have values presented in, for example, The Handbook of Chemistry and Physics. For liquids that are not described in this handbook, the values presented in WO2014/126034 may be used. For liquids that are not described in either of these publications, the surface tension can be determined following a known measurement method, for example, such as the ring method or the Wilhelmy method. Preferably, the surface tension at 25°C is measured with the automatic surface tensiometer CBVP-Z manufactured by Kyowa Interface Science Co., Ltd., or SIGMA 702 manufactured by KSV INSTRUMENTS LTD (currently, Biolin Scientific AB).

The liquid may be an organic solvent having the foregoing ranges of surface tensions. The organic solvent may be any of the aforementioned organic solvents with the foregoing ranges of surface tensions. However, for considerations such as the capability to inhibit particle aggregation during drying and provide a denser zirconia sintered body, the organic solvent is preferably at least one selected from the group consisting of methanol, ethanol, 2-methoxyethanol, 1,4-dioxane, 2-ethoxyethanol, and 2-(2-ethoxyethoxy)ethanol, more preferably at least one selected from the group consisting of methanol, ethanol, 2-ethoxyethanol, and 2-(2-ethoxyethoxy)ethanol.

For considerations such as the capability to inhibit particle aggregation during drying and provide a denser zirconia sintered body, the liquid content in the dispersion medium is preferably 50 mass% or more, more preferably 80 mass% or more, even more preferably 95 mass% or more, particularly preferably 99 mass% or more.

A slurry employing a dispersion medium other than water can be obtained by replacing the dispersion medium of a slurry dispersed in water. The method used to replace the dispersion medium is not particularly limited, and may be, for example, a method that removes water by distillation after adding a dispersion medium other than water (e.g., an organic solvent) to a slurry dispersed in water. For removal of water by distillation, the dispersion medium other than water may be distilled away with water, either partially or entirely. The addition of a dispersion medium other than water, and the removal of water by distillation may be repeated multiple times. As an alternative method, the dispersoid may be precipitated after adding a dispersion medium other than water to a slurry dispersed in water. Alternatively, the dispersion medium after replacement with a specific organic solvent in a slurry dispersed in water may be replaced with yet another organic solvent.

The fluorescent agent may be added after replacing the dispersion medium. However, for considerations such as the capability to provide a more uniform zirconia sintered body of even superior properties, it is preferable to add the fluorescent agent before replacing the dispersion medium. Similarly, when containing a colorant and/or a translucency adjuster in the slurry, these may be added after replacing the dispersion medium. However, for considerations such as the capability to provide a more uniform zirconia sintered body of even superior properties, it is preferable to add the colorant and/or translucency adjuster before replacing the dispersion medium.

Examples of the building-up process include:

the vapor-phase pyrolysis method, which involves the precipitation of oxides through the pyrolysis of oxoacid salts of metal ions or organometallic compounds with high vapor pressure during vaporization;
the vapor-phase reaction method, where synthesis occurs through a vapor-phase chemical reaction between a gas of a high-vapor-pressure metal compound and a reactant gas;
the evaporative concentration method, which involves vaporizing the raw materials by heating and then condensing the vapor into fine particles through rapid cooling in an inert gas at a predetermined pressure;
the melt method, which involves cooling and solidifying the melt into small droplets to form a powder;
the solvent evaporation method, which evaporates the solvent to increase the concentration in the liquid to a supersaturated state to cause precipitation; and
the precipitation method, which involves supersaturating the solute concentration through a reaction with a precipitant or hydrolysis, and precipitating poorly soluble compounds such as oxides and hydroxides through nucleation and growth processes.

The precipitation method can be further divided into the following methods:

the homogenous precipitation method, which involves the formation of a precipitant in the solution through a chemical reaction to eliminate local non-uniformity in precipitant concentration;
the coprecipitation method, which involves adding a precipitant to cause simultaneous precipitation of multiple metal ions coexisting in the solution;
the hydrolysis method, which produces oxides or hydroxides through hydrolysis from metal salt solutions or alcohol solutions such as metal alkoxides; and

the solvothermal synthesis method, which produces oxides or hydroxides from high-temperature and high-pressure fluids.

The solvothermal synthesis method is subdivided into methods such as the hydrothermal synthesis method that utilizes water as a solvent, and the supercritical synthesis method that employs a supercritical fluid, such as water or carbon dioxide, as a solvent.

In the present invention, in view of providing the desired average particle diameters (r1) and (r2) for zirconia powder and stabilizer powder, it is preferable not to employ the coprecipitation method with zirconia and stabilizer.

The zirconium source in the building-up process may be, for example, nitrates, acetates, chlorides, or alkoxides. Specific examples of the zirconium source include zirconium oxychloride, zirconium acetate, and zirconyl nitrate.

When produced by a method such as the building-up process, the zirconia powder may be used in the form of a zirconia particle-containing slurry, without drying, in the mixing process of zirconia powder and stabilizer powder, provided that the zirconia particles satisfy the desired range of average particle diameters. The slurry containing zirconia particles may be prepared using any methods. For example, the slurry may be one obtained through the breakdown process or building-up process.

When a zirconia composition of the present invention is a dry zirconia composition, it can be obtained by drying the slurry containing zirconia particles.

A zirconia composition of the present invention may be formed into a molded body through a molding process.

The term "molded body" means a formed body that has not been brought to a semi-sintered (pre-sintered) or sintered state. That is, "molded body" distinguishes itself from pre-sintered body and sintered body in that it is a formed body that has not been fired after molding.

The molding process is not particularly limited. However, for considerations such as ease of production of a zirconia molded body of the present invention, and, in turn, a zirconia pre-sintered body and zirconia sintered body of the present invention, the molding process is preferably at least one of the following processes:

(i) slip casting of a slurry containing zirconia-based particles
(ii) gel casting of a slurry containing zirconia-based particles
(iii) press forming of a powder containing zirconia-based particles
(iv) molding of a composition containing zirconia-based particles and resin
(v) polymerization of a composition containing zirconia-based particles and a polymerizable monomer or oligomer
(vi) additive fabrication of granules containing zirconia-based particles

More preferably, the molding process is a process that includes molding of zirconia-based particles, a polyol, and a binder to obtain a zirconia molded body.

(i) Slip Casting

When the zirconia molded body is produced using a method that includes the step of slip casting a slurry containing zirconia-based particles, the slip casting method is not particularly limited to specific methods, and may be, for example, a method that dries the zirconia-based particle-containing slurry after pouring it in a mold.

For considerations such as ease of pouring the slurry into a mold, and increasing the reusability of the mold in addition to preventing excessive drying time, the content of the dispersion medium in the zirconia-based particle-containing slurry used is preferably 80 mass% or less, more preferably 50 mass% or less, even more preferably 20 mass% or less.

The slurry may be poured into a mold under ordinary pressure. However, in view of production efficiency, it is preferable to pour the slurry under applied pressure conditions. The mold used for slip casting is not particularly limited, and the mold may be, for example, a porous mold made of a material such as plaster, resin, or ceramic. Resin or ceramic porous molds are superior in terms of durability.

The zirconia-based particle-containing slurry used for slip casting may contain one additional component or two or more additional components, such as the binders, plasticizers, dispersants, emulsifiers, antifoaming agents, pH adjusters, and lubricants mentioned earlier.

(ii) Gel Casting

When a zirconia molded body is produced using a method that includes the step of gel casting a slurry containing zirconia-based particles, the gel casting method is not particularly limited to specific methods, and may be, for example, a method that dries a moist body shaped in a mold by gelling a slurry containing zirconia-based particles and a fluorescent agent.

For considerations such as preventing excessive drying time and reducing cracking during drying, the content of the dispersion medium contained in the zirconia-based particle-containing slurry used is preferably 80 mass% or less, more preferably 50 mass% or less, even more preferably 20 mass% or less.

The gelation may be achieved by, for example, adding a gelatinizer, or by adding and polymerizing a polymerizable monomer. The mold used is not particularly limited, and may be, for example, a porous mold made of a material such as plaster, resin, or ceramic, or a non-porous mold made of a material such as metal or resin.

The gelatinizer is not particularly limited, and may be, for example, a water-soluble gelatinizer. Specifically, preferred are agarose and gelatin, for example. The gelatinizer may be used alone, or two or more thereof may be used in combination. The gelatinizer may be used in any amount, as long as it does not cause problems such as cracking during sintering. The gelatinizer may be used in an amount of 10 mass% or less, 5 mass% or less, or 1 mass% or less based on the mass the slurry after the addition of gelatinizer.

The polymerizable monomer is not particularly limited, and may be, for example, (meth)acrylate monomers such as 2-hydroxyethyl (meth)acrylate, 3-hydroxypropyl (meth)acrylate, 4-hydroxybutyl (meth)acrylate, 6-hydroxyhexyl (meth) acrylate, 10-hydroxydecyl (meth)acrylate, propylene glycol mono(meth)acrylate, glycerol mono(meth)acrylate, and erythritol mono(meth)acrylate; and (meth)acrylamide monomers such as N-methylol(meth)acrylamide, N-hydroxyethyl(meth)acrylamide, and N,N-bis(2-hydroxyethyl)(meth)acrylamide.

The polymerizable monomer may be used alone, or two or more thereof may be used in combination.

The polymerizable monomer may be used in any amount, as long as it does not cause problems such as cracking during sintering. The polymerizable monomer may be used in an amount of 10 mass% or less, 5 mass% or less, or 1 mass% or less based on the mass the slurry after the addition of polymerizable monomer.

When gelation is performed through polymerization of a polymerizable monomer, it is preferable to use a polymerization initiator for polymerization. The polymerization initiator is not particularly limited. Particularly preferred are photopolymerization initiators. A photopolymerization initiator can be appropriately selected from photopolymerization initiators used in industry, particularly those used in dentistry.

Specific examples of the photopolymerization initiators include (bis)acylphosphine oxides (including salts), thioxanthones (including salts such as quaternary ammonium salts), ketals, $\alpha$-diketones, coumarins, anthraquinones, benzoin alkyl ether compounds, and $\alpha$-aminoketone compounds. The photopolymerization initiators may be used alone, or two or more thereof may be used in combination. Preferred among these photopolymerization initiators is at least one selected from the group consisting of a (bis)acylphosphine oxide and an $\alpha$-diketone. With these photopolymerization initiators, it is possible to achieve polymerization (gelation) both in the ultraviolet region (including the near ultraviolet region) and the visible region. Specifically, polymerization (gelation) can sufficiently take place irrespective of whether the light source used is a laser such as an Ar laser or a He-Cd laser, or a lamp such as a halogen lamp, a xenon lamp, a metal halide lamp, a light emitting diode (LED), a mercury lamp, or a fluorescent lamp.

Examples of acylphosphine oxides in the (bis)acylphosphine oxides include 2,4,6-trimethylbenzoyldiphenylphosphine oxide (commonly known as TPO), 2,6-dimethoxybenzoyldiphenylphosphine oxide, 2,6-dichlorobenzoyldiphenylphosphine oxide, 2,4,6-trimethylbenzoylmethoxyphenylphosphine oxide, 2,4,6-trimethylbenzoylethoxyphenylphosphine oxide, 2,3,5,6-tetramethylbenzoyldiphenylphosphine oxide, benzoyl di(2,6-dimethylphenyl)phosphonate, sodium salts of 2,4,6-trimethylbenzoylphenylphosphine oxide, potassium salts of 2,4,6-trimethylbenzoyldiphenylphosphine oxide, and ammonium salts of 2,4,6-trimethylbenzoyldiphenylphosphine oxide.

Examples of bisacylphosphine oxides in the (bis)acylphosphine oxides include bis(2,6-dichlorobenzoyl)phenylphosphine oxide, bis(2,6-dichlorobenzoyl)-2,5-dimethylphenylphosphine oxide, bis(2,6-dichlorobenzoyl)-4-propylphenylphosphine oxide, bis(2,6-dichlorobenzoyl)-1-naphthylphosphine oxide, bis(2,6-dimethoxybenzoyl)phenylphosphine oxide, bis(2,6-dimethoxybenzoyl)-2,4,4-trimethylpentylphosphine oxide, bis(2,6-dimethoxybenzoyl)-2,5-dimethylphenylphosphine oxide, bis(2,4,6-trimethylbenzoyl)phenylphosphine oxide, and bis(2,3,6-trimethylbenzoyl)-2,4,4-trimethylpentylphosphine oxide. Other examples include compounds described in JP 2000-159621 A.

Preferred among these (bis)acylphosphine oxides are 2,4,6-trimethylbenzoyldiphenylphosphine oxide, 2,4,6-trimethylbenzoylmethoxyphenylphosphine oxide, bis(2,4,6-trimethylbenzoyl)phenylphosphine oxide, and sodium salts of 2,4,6-trimethylbenzoylphenylphosphine oxide.

Examples of the $\alpha$-diketones include diacetyl, benzyl, camphorquinone, 2,3-pentadione, 2,3-octadione, 9,10-phenanthrenequinone, 4,4'-oxybenzyl, and acenaphthenequinone. Preferred is camphorquinone, particularly in situations where a visible light source is used.

The zirconia-based particle-containing slurry used for gel casting may additionally contain one additional component or two or more additional components, such as the binders, plasticizers, dispersants, emulsifiers, antifoaming agents, pH adjusters, and lubricants mentioned earlier, as with the case of the slurry used for slip casting.

The method used to dry the shaped moist body is not particularly limited, and may be, for example, natural drying, hot-air drying, vacuum drying, drying by dielectric heating, drying by induction heating, or drying under constant temperature and humidity. These methods may be used alone, or two or more thereof may be used in combination.

In view of considerations such as the capability to reduce cracking during drying, preferred are natural drying, drying by dielectric heating, drying by induction heating, and drying under constant temperature and humidity.

(iii) Press Forming

When the zirconia molded body is produced using a method that comprises the step of press forming a powder containing zirconia-based particles, the press forming method is not particularly limited to specific methods, and a known press forming machine can be used.

Specific examples of the pressing forming method include uniaxial pressing.

In press forming, the applied pressure is appropriately optimized according to factors such as the desired size, open porosity, and biaxial flexural strength of the molded body, and the particle size of the raw material powder. Typically, the pressure ranges from 5 MPa to 1,000 MPa. In the production method described above, higher pressures during molding lead to more pore closure in the resulting molded body, enabling lower open porosities to be set for the molded body and achieving higher density in the molded body. In order to increase the density of the zirconia molded body obtained, the uniaxial pressing may be followed by a cold isostatic pressing (CIP) process.

The zirconia-based particle-containing powder used for press forming may additionally contain one additional component or two or more additional components, such as the binders, plasticizers, dispersants, emulsifiers, antifoaming agents, pH adjusters, lubricants, and translucency adjusters mentioned earlier. These components may be added during the preparation of the powder.

(iv) Molding of Resin-Containing Composition

When the zirconia molded body is produced using a method that comprises the step of molding a composition containing zirconia-based particles and resin, the method used to mold the composition is not particularly limited to specific methods, and may be, for example, injection molding, cast molding, or extrusion molding. It is also possible to use additive fabrication techniques (such as 3D printing) to form the composition, including, for example, fused deposition modeling (FDM), the inkjet method, and the powder/binder lamination method. Preferred among these molding methods are injection molding and cast molding, more preferably injection molding.

The resin is not particularly limited, and is preferably one that can serve as a binder. Specific examples of the resin include paraffin wax, polyvinyl alcohol, polyethylene, polypropylene, an ethylene-vinyl acetate copolymer, polystyrene, atactic polypropylene, methacrylic resin, and fatty acids such as stearic acid. These resins may be used alone, or two or more thereof may be used in combination.

The composition containing zirconia-based particles and resin may additionally contain one additional component or two or more additional components, such as the plasticizers, dispersants, emulsifiers, antifoaming agents, pH adjusters, and lubricants mentioned earlier.

(v) Polymerization of Composition Containing Polymerizable Monomer or Oligomer

By polymerizing a composition containing zirconia-based particles along with polymerizable monomer or oligomer, the polymerizable monomer in the composition can polymerize, allowing the composition to cure.

When the zirconia molded body is produced using a method that comprises such a polymerization step, the method is not particularly limited to specific methods, and may be, for example, (a) a method by which a composition containing zirconia-based particles and polymerizable monomer or oligomer is polymerized in a mold, or (b) stereolithography (SLA) using a composition containing zirconia-based particles and polymerizable monomer or oligomer. The preferred method is (b), stereolithography.

Stereolithography enables the zirconia molded body to have a shape corresponding to the shape desired for the final zirconia sintered body, at the time of its production. This makes stereolithography a potentially preferred method in certain situations, particularly when a zirconia sintered body of the present invention is used as a dental material such as a dental prosthesis.

The polymerizable monomer in the composition containing zirconia-based particles and polymerizable monomer or oligomer is not particularly limited, and may be a monofunctional polymerizable monomer such as a monofunctional (meth)acrylate or a monofunctional (meth)acrylamide, or a polyfunctional polymerizable monomer such as a bifunctional aromatic compound, a bifunctional aliphatic compound, or a tri- or higher-functional compound. The polymerizable monomer may be used alone, or two or more thereof may be used in combination.

The oligomer is not particularly limited, as long as it is a polymerizable compound formed by the bonding of two or more of the aforementioned polymerizable monomers.

Preferred for use are polyfunctional polymerizable monomers, particularly in situations where stereolithography is employed.

Examples of the monofunctional (meth)acrylate include:

(meth)acrylates having a hydroxyl group, such as 2-hydroxyethyl (meth)acrylate, 2-hydroxypropyl (meth)acrylate, 3-hydroxypropyl (meth)acrylate, 4-hydroxybutyl (meth)acrylate, 6-hydroxyhexyl (meth)acrylate, 10-hydroxydecyl (meth)acrylate, propylene glycol mono(meth)acrylate, glycerol mono(meth)acrylate, and erythritol mono(meth)acrylate;
alkyl (meth)acrylates such as methyl (meth)acrylate, ethyl (meth)acrylate, propyl (meth)acrylate, isopropyl (meth)acrylate, n-butyl (meth)acrylate, sec-butyl (meth)acrylate, t-butyl (meth)acrylate, isobutyl (meth)acrylate, n-hexyl (meth)acrylate, lauryl (meth)acrylate, cetyl (meth)acrylate, and stearyl (meth)acrylate;
alicyclic (meth)acrylates such as cyclohexyl (meth)acrylate, and isobornyl (meth)acrylate;
aromatic group-containing (meth)acrylates such as benzyl (meth)acrylate, and phenyl (meth)acrylate; and
(meth)acrylates having a functional group, such as 2,3-dibromopropyl (meth)acrylate, 3-(meth)acryloyloxypropyltrimethoxysilane, and 11-(meth)acryloyloxyundecyltrimethoxysilane.

Examples of the monofunctional (meth)acrylamide include (meth)acrylamide, N-(meth)acryloylmorpholine, N,N-dimethyl(meth)acrylamide, N,N-diethyl(meth)acrylamide, N,N-di-n-propyl(meth)acrylamide, N,N-di-n-butyl(meth)acrylamide, N,N-di-n-hexyl(meth)acrylamide, N,N-di-n-octyl(meth)acrylamide, N,N-di-2-ethylhexyl(meth)acrylamide, N-hydroxyethyl(meth)acrylamide, and N,N-bis(2-hydroxyethyl)(meth)acrylamide.

In view of excellent polymerizability, preferred among these monofunctional polymerizable monomers are (meth)acrylamide, more preferably N-(meth)acryloylmorpholine, N,N-dimethyl(meth)acrylamide, and N,N-diethyl(meth)acrylamide.

Examples of the bifunctional aromatic compound include (meth)acrylates such as 2,2-bis((meth)acryloyloxyphenyl)propane, 2,2-bis[4-(2-hydroxy-3-acryloyloxypropoxy)phenyl]propane, 2,2-bis[4-(2-hydroxy-3-methacryloyloxypropoxy)phenyl]propane (commonly known as Bis-GMA), 2,2-bis(4-(meth)acryloyloxyethoxyphenyl)propane, 2,2-bis(4-(meth)acryloyloxypolyethoxyphenyl)propane, 2,2-bis(4-(meth)acryloyloxydiethoxyphenyl)propane, 2,2-bis(4-(meth)acryloyloxytetraethoxyphenyl)propane, 2,2-bis(4-(meth)acryloyloxypentaethoxyphenyl)propane, 2,2-bis(4-(meth)acryloyloxydipropoxyphenyl)propane, 2-(4-(meth)acryloyloxydiethoxyphenyl)-2-(4-(meth)acryloyloxyethoxyphenyl)propane, 2-(4-(meth)acryloyloxydiethoxyphenyl)-2-(4-(meth)acryloyloxytriethoxyphenyl)propane, 2-(4-(meth)acryloyloxydipropoxyphenyl)-2-(4-(meth)acryloyloxytriethoxyphenyl)propane, 2,2-bis(4-(meth)acryloyloxypropoxyphenyl)propane, 2,2-bis(4-(meth)acryloyloxyisopropoxyphenyl)propane, and 1,4-bis(2-(meth)acryloyloxyethyl)pyromellitate. In view of excellent polymerizability and providing a zirconia molded body having superior mechanical strength, preferred are Bis-GMA, and 2,2-bis(4-(meth)acryloyloxypolyethoxyphenyl)propane. Preferred as 2,2-bis(4-(meth)acryloyloxypolyethoxyphenyl)propane is 2,2-bis(4-methacryloyloxypolyethoxyphenyl)propane (with an average of 2.6 moles of ethoxy group added; commonly known as D-2.6E).

Examples of the bifunctional aliphatic compound include (meth)acrylates such as glycerol di(meth)acrylate, ethylene glycol di(meth)acrylate, diethylene glycol di(meth)acrylate, triethylene glycol di(meth)acrylate, propylene glycol di(meth)acrylate, butylene glycol di(meth)acrylate, neopentyl glycol di(meth)acrylate, polyethylene glycol di(meth)acrylate, 1,3-butanediol di(meth)acrylate, 1,4-butanediol di(meth)acrylate, 1,6-hexanediol di(meth)acrylate, 2-ethyl-1,6-hexanediol di(meth)acrylate, 1,9-nonanediol di(meth)acrylate, 1,10-decanediol di(meth)acrylate, 1,2-bis(3-methacryloyloxy-2-hydroxypropoxy)ethane, 2,2,4-trimethylhexamethylenebis(2-carbamoyloxyethyl)dimethacrylate (commonly known as UDMA). In view of excellent polymerizability and providing a zirconia molded body having superior mechanical strength, preferred are triethylene glycol dimethacrylate (commonly known as TEGDMA), and 2,2,4-trimethylhexamethylenebis(2-carbamoyloxyethyl)dimethacrylate.

Examples of the tri- or higher-functional compound include (meth)acrylates such as trimethylolpropane tri(meth)acrylate, trimethylolethane tri(meth)acrylate, trimethylolmethane tri(meth)acrylate, pentaerythritol tri(meth)acrylate, pentaerythritol tetra(meth)acrylate, dipentaerythritol penta(meth)acrylate, N,N-(2,2,4-trimethylhexamethylene)bis[2-(aminocarboxy)propane-1,3-diol]tetra(meth)acrylate, and 1,7-diacryloyloxy-2,2,6,6-tetra(meth)acryloyloxymethyl-4-oxaheptane. In view of excellent polymerizability and providing a zirconia molded body having superior mechanical strength, preferred are N,N-(2,2,4-trimethylhexamethylene)bis[2-(aminocarboxy)propane-1,3-diol]tetramethacrylate, and 1,7-diacryloyloxy-2,2,6,6-tetraacryloyloxymethyl-4-oxaheptane.

In both methods (a) and (b), it is preferable that the composition be polymerized using a polymerization initiator, and that the composition additionally comprise a polymerization initiator. The polymerization initiator is not particularly limited. Particularly preferred are photopolymerization initiators. A photopolymerization initiator may be appropriately selected from photopolymerization initiators used in industry, preferably those used in dentistry. Specific examples of the photopolymerization initiators are no different from those mentioned above in the description of gel casting, and repetition is omitted.

The composition containing zirconia-based particles and polymerizable monomer may additionally contain one additional component or two or more additional components, such as the plasticizers, dispersants, emulsifiers, antifoaming agents, pH adjusters, and lubricants mentioned earlier.

When the zirconia molded body is produced by stereolithography using a composition containing zirconia-based particles and polymerizable monomer, the stereolithography method is not particularly limited to specific methods, and a known method may be appropriately selected for stereolithography. For example, it is possible to use a method that produces the desired zirconia molded body by successively forming layers in desired shapes through photopolymerization of a liquid composition with, for example, ultraviolet light or a laser, using a stereolithography device.

When the zirconia molded body is produced by stereolithography, it is preferable that the composition containing zirconia-based particles and polymerizable monomer have the highest possible content of zirconia-based particles, in view of considerations such as sinterability in a later step. Specifically, the content of the zirconia-based particles in the composition is preferably 20 mass% or more, more preferably 30 mass% or more, even more preferably 40 mass% or more, particularly preferably 50 mass% or more. In stereolithography, it is preferable for the viscosity of the composition to fall in a certain range, for reasons related to the principles of layer-to-layer formation. In this respect, the content of the zirconia-based particles in the composition is preferably 90 mass% or less, more preferably 80 mass% or less, even more preferably 70 mass% or less, particularly preferably 60 mass% or less. Adjusting the viscosity of the composition can be particularly important when implementing the constrained surface method, which involves curing layers by applying light through the bottom of a vat from its lower side to successively form layers of zirconia molded body, in order to enable the composition to form a new layer by smoothly flowing into the space between the bottom surface of the cured layer and the bottom of the vat after the cured layer is elevated by one layer thickness.

Specifically, the composition has a viscosity of preferably 20,000 mPa·s or less, more preferably 10,000 mPa·s or less, even more preferably 5,000 mPa·s or less in terms of a viscosity at 25°C. The viscosity is preferably 100 mPa·s or more. Because the composition tends to increase its viscosity with increasing content of zirconia particles, it is preferable to appropriately adjust the balance between zirconia particle content and viscosity in the composition, taking into consideration a balance between the rate of stereolithography and the precision of the zirconia molded body produced, depending on factors such as the capabilities of the stereolithography device used. The viscosity can be measured with an E-type viscometer.

In a method of production of a zirconia molded body of the present invention, the zirconia molded body may be subjected to a CIP process after a humidification process, in order to further improve the density of the zirconia molded body.

When press forming is performed, the zirconia particle-containing powder may be subjected to press forming after a humidification process, before pressing the powder. Any known methods can be used for the humidification process, including a method that sprays water with a sprayer or the like, and a method that uses a constant humidity chamber or a constant temperature and humidity chamber. The amount of moisture increase after the humidification process is preferably higher than 2 mass%, more preferably higher than 3 mass%, even more preferably higher than 4 mass%, particularly preferably higher than 5 mass%, and is preferably 15 mass% or less, more preferably 13 mass% or less, even more preferably 11 mass% or less relative to the mass of the unwetted powder (powder before humidification process) and molded body, though the amount of moisture increase depends on factors such as the particle size of the zirconia particles and stabilizer particles contained. The amount of moisture increase by humidification process can be determined as a percentage by subtracting the mass of the unwetted powder and molded body from the mass of the wetted powder (powder after humidification process) and molded body, and dividing the difference by the mass of the unwetted powder and molded body.

The CIP process uses the same pressure as specified previously for press forming.

(vi) Process for Additive Fabrication of Granules Containing Zirconia-Based Particles

There is no specific restriction on the method for producing granules containing zirconia-based particles. For example, granules can be obtained by preparing a slurry and drying it with a spray dryer, and used for powder additive fabrication.

The technique employed for powder additive fabrication is not particularly limited. Examples include the powder bed method, SLS method (selective laser sintering), SLM method (selective laser melting), electron beam method, arc discharge method, and binderr jet method. For techniques where organic materials should be excluded in additive fabrication, it is preferable to avoid using organic materials during the production of granules.

[Zirconia Sintered Body]

The following describes a zirconia sintered body of the present invention. A zirconia sintered body of the present invention can be produced using the zirconia pre-sintered body. Specifically, a zirconia sintered body of the present invention is obtained by, for example, sintering the zirconia pre-sintered body.

The zirconia sintered body is in a fully sintered state, where the zirconia particles have consolidated as a result of sintering, and the sintering process has led to an increase in relative density, with the progression of densification leading to a relative density of 95% or more.

The stabilizer content specified for a zirconia pre-sintered body of the present invention is also applicable to a zirconia sintered body of the present invention.

A zirconia sintered body of the present invention satisfies the formula (1) above when the first translucency $\Delta L_1^*$ (W-B) of a zirconia sintered body fabricated by sintering at 1,550°C for 120 minutes is compared to the second translucency $\Delta L_2^*$(W-B) of a zirconia sintered body fabricated by sintering at 1,550°C for 10 minutes.

Consequently, a zirconia sintered body of the present invention can maintain high translucency comparable to that achieved by extended sintering, even after short sintering with a hold time of 10 minutes or less at the highest sintering temperature.

A zirconia sintered body of the present invention may comprise a fluorescent agent. The fluorescent agent may be the same fluorescent agent used in the zirconia pre-sintered body. A zirconia sintered body of the present invention may comprise a single fluorescent agent by itself, or may comprise two or more fluorescent agents in combination.

In this specification, concerning the fluorescent agent, the phrase "relative to 100 mass% of zirconia contained in zirconia pre-sintered body" can be read as "relative to 100 mass% of zirconia contained in zirconia sintered body".

A zirconia sintered body of the present invention may comprise a colorant. The colorant may be the same colorant used in the zirconia pre-sintered body.

The colorant content in the zirconia sintered body is not particularly limited, and may be appropriately adjusted according to factors such as the type of colorant, and intended use of the zirconia sintered body. In view of considerations such as suitability in dental prosthesis applications, the colorant content is preferably 0.001 mass% or more, more preferably 0.005 mass% or more, even more preferably 0.01 mass% or more in terms of an oxide of a metallic element contained in the colorant, relative to 100 mass% of zirconia contained in the zirconia sintered body. The colorant content is preferably 5 mass% or less, more preferably 1 mass% or less, even more preferably 0.5 mass% or less in terms of an oxide of a metallic element contained in the colorant. The colorant content may be 0.1 mass% or less, or 0.05 mass% or less.

A zirconia sintered body of the present invention may comprise a translucency adjuster, in order to adjust the translucency in the zirconia sintered body. The translucency adjuster may be the same translucency adjuster used in the zirconia pre-sintered body.

The content of the translucency adjuster in the zirconia sintered body is not particularly limited, and may be appropriately adjusted according to factors such as the type of translucency adjuster, and intended use of the zirconia sintered body. In view of considerations such as suitability in dental prosthesis applications, the content of the translucency adjuster is preferably 0.1 mass% or less relative to 100 mass% of zirconia contained in the zirconia sintered body.

[Production Method of Zirconia Sintered Body]

A zirconia sintered body of the present invention can be produced by, for example, a method that sinters the zirconia pre-sintered body. Preferably, the method comprises the step of sintering the zirconia pre-sintered body at more than 900°C and 1,700°C or less under ordinary pressure. In this way, the method can easily produce a zirconia sintered body of the present invention, where high translucency comparable to that achieved by extended sintering can be maintained even after short sintering with a hold time of 10 minutes or less at the highest sintering temperature.

A zirconia sintered body of the present invention can also be produced by sintering a zirconia pre-sintered body of the present invention under ordinary pressure.

Preferably, the sinterable temperature (for example, highest sintering temperature) conditions are chosen to achieve maximum translucency in the zirconia sintered body when the sintered body is produced by sintering a zirconia pre-sintered body of the present invention.

In view of considerations such as ease of obtaining the intended zirconia sintered body, the sinterable temperature is preferably more than 900°C, more preferably 1,000°C or more, even more preferably 1,050°C or more, and is preferably 1,700°C or less, more preferably 1,650°C or less, even more preferably less than 1,600°C under ordinary pressure.

In view of achieving superior translucency in short sintering with lower sinterable temperatures, a certain preferred embodiment of the production method of zirconia sintered body sinters the zirconia pre-sintered body at more than 900°C and 1,560°C or less under ordinary pressure. This is industrially more advantageous because superior translucency can be achieved in short sintering at lower sinterable temperatures, compared to related art.

When the sinterable temperature is at or above the foregoing lower limits and at or below the upper limits specified above, the sintering process can sufficiently proceed, easily yielding a dense sintered body. When the sinterable temperature is at or below the foregoing upper limits, it is possible to inhibit deactivation of the fluorescent agent.

In producing the sintered body, the sintering time is not particularly limited, as long as the hold time at the highest sintering temperature is 10 minutes or less. However, for considerations such as enabling efficient and stable production of the intended zirconia sintered body with good productivity, the hold time at the sinterable temperature (for example, the highest sintering temperature) is preferably 10 minutes or less, more preferably 9 minutes or less, even more preferably 8 minutes or less, yet more preferably 7 minutes or less, particularly preferably 6 minutes or less, most preferably 5 minutes or less. The hold time is preferably 1 minute or more, more preferably 2 minutes or more, even more preferably 3 minutes or more.

In producing the sintered body, a shorter sintering time can be employed for the fabrication of the sintered body without causing a decrease in the translucency of the zirconia sintered body prepared. Particularly, the hold time at the highest sintering temperature can be reduced to 10 minutes or less for the fabrication of the sintered body. This can lead to enhanced production efficiency, and, when a zirconia pre-sintered body of the present invention is used for dental products, the patient can experience less time-related stress because it takes a shorter time before a treatment can be started with a dental product after its dimensions are determined and the product is milled for the treatment. It is also possible to reduce the energy cost.

Preferably, the rate of temperature increase and the rate of temperature decrease in the sintering process are set so as to reduce the time required for the sintering process. For example, the rate of temperature increase may be set so that the highest sintering temperature is reached in the shortest possible time, depending on the capabilities of the furnace. The rate of temperature increase to the highest sintering temperature may be, for example, 10°C/min or more, 50°C/min or more, 100°C/min or more, 120°C/min or more, 150°C/min or more, or 200°C/min or more. Preferably, the rate of temperature decrease is set to a rate that does not cause defects, such as cracks, in the sintered body. For example, the sintered body may be allowed to cool at room temperature after the heating is finished.

In the present invention, a sintering furnace can be used for sintering. The type of sintering furnace is not particularly limited, and may be a sintering furnace used in industry, for example, such as an electric furnace or debinding furnace. Specifically, when the intended use is dental material applications, it is possible to use a dental porcelain furnace, which involves a relatively low sinterable temperature (for example, highest sintering temperature), aside from conventional dental sintering furnaces for zirconia.

A zirconia sintered body of the present invention can be produced with ease, even without a HIP process. However, the translucency and mechanical strength can further improve when a HIP process follows the sintering performed under ordinary pressure.

[Uses of Zirconia Sintered Body]

A zirconia sintered body of the present invention is not limited to particular uses.

However, because a zirconia sintered body of the present invention excels in translucency and linear light transmittance, a zirconia sintered body of the present invention is particularly suited for applications such as dental prostheses and other dental materials, and is highly useful not only as a dental prosthesis used for the cervical region but also as a dental prosthesis used for the occlusal surface of a posterior tooth, or the incisal region of a front tooth.

Particularly, a zirconia sintered body of the present invention is highly useful as a dental prosthesis used for the incisal region of a front tooth.

The present invention encompasses embodiments combining the foregoing features, provided that the present invention can exhibit its effects with such combinations made in various forms within the technical idea of the present invention.

EXAMPLES

The present invention is described below in greater detail through Examples. It is to be noted, however, that the present invention is in no way limited by the following Examples, and various modifications may be made by a person with ordinary skill in the art within the technical idea of the present invention.

[Raw Material Powder]

Monoclinic crystal 0Y: zirconia powder (TZ-0 manufactured by Tosoh Corporation)
Tetragonal crystal 3Y: zirconia powder containing 3 mol% yttria in solid solution (TZ-3Y-E manufactured by Tosoh Corporation)
Cubic crystal 6Y: zirconia powder containing 6 mol% yttria in solid solution (TZ-6Y manufactured by Tosoh Corporation)
Cubic crystal 10Y: zirconia powder containing 10 mol% yttria in solid solution (TZ-10Y manufactured by Tosoh Corporation)
Cubic crystal 15Y: A hydrous zirconia sol obtained through the hydrolysis reaction of an aqueous solution of zirconium oxychloride was mixed with yttria and subjected to drying, and the resulting powder was heat treated in the atmosphere at 1,160°C for 2 hours to yield a zirconia powder containing 15 mol% yttria.
Yttria: yttria powder ($Y_2O_3$ manufactured by Treibacher Industrie AG)

<Examples 1 to 15 and Comparative Examples 1 to 6>

[Preparation of Zirconia Compositions of Examples 1 to 15 and Comparative Examples 1 to 3]

In order to prepare a granular raw material composition for each Example and Comparative Example, the raw material powders above were mixed according to the formulations specified in Table 1. The mixture was then subjected to wet pulverization and mixing for 20 hours with added water, using a ball mill. After pulverization, a binder was added into the resulting slurry, which was then dried with a spray dryer to obtain a granular zirconia composition.
In Tables 1 and 2 below, "total yttria content" indicates the content of yttria relative to the total mole of zirconia and yttria.

[Preparation of Zirconia Compositions of Comparative Examples 4 and 5]

The raw material powders above were mixed according to the formulations specified in Table 1. The mixture was then subjected to wet pulverization and mixing for 20 hours with added water, using a ball mill. After pulverization, a binder was added into the resulting slurry, which was then dried with a spray dryer to obtain a granule. The granule was then heated to 1,000°C at 10°C/min. After being retained for 2 hours, the granule was subjected to wet pulverization and mixing for 20 hours with added water. After pulverization, a binder was added into the resulting slurry, which was then dried with a spray dryer to obtain a granular zirconia composition.

[Preparation of Zirconia Composition of Comparative Example 6]

The monoclinic crystal 0Y powder and the tetragonal crystal 3Y powder were mixed according to the formulation specified in Table 1, and the resulting mixture was subjected to wet pulverization and mixing for 15 hours with added water, using a ball mill.
This slurry was then mixed with the yttria powder according to the formulation specified in Table 1, and subjected to wet pulverization and mixing for 5 hours with added water using a ball mill. After pulverization, a binder was added into the slurry, which was then dried with a spray dryer to obtain a granular zirconia composition.

[Preparation of Zirconia Pre-Sintered Body]

For each Example and Comparative Example, pellet-shaped pre-sintered bodies were produced in preparation for the fabrication of zirconia sintered body samples for translucency evaluation, as follows.
The raw material composition was placed in a cylindrical mold with a diameter of 19 mm to ensure that sintering produces a processable zirconia composite sintered body with a thickness of 1.2 mm. Subsequently, the raw material composition was molded under a surface pressure of 200 MPa to obtain a pellet-shaped molded body, using a uniaxial press molding machine. The pellet-shaped molded body was then heated to 1,000°C at 10°C/min using the Noritake KATANA® F-1 furnace manufactured by SK Medical Electronics Co., Ltd. After being retained for 2 hours, the molded body was cooled to obtain a zirconia pre-sintered body.

[Preparation of Zirconia Sintered Body]

The pellet-shaped pre-sintered body was used to prepare samples for the measurement of $\Delta L_1{}^*$(W-B), measured for first sintered bodies fabricated by sintering at 1,550°C for 120 minutes, and $\Delta L_2{}^*$(W-B), measured for second sintered bodies fabricated by sintering at 1,550°C for 10 minutes, using the Noritake KATANA® F-1 furnace manufactured by SK Medical Electronics Co., Ltd.
The zirconia sintered bodies produced for each Example and Comparative Example were evaluated for their properties using the methods below.

<Measurement Method for Average Primary Particle Diameter of Particles in Zirconia Composition>

The particles (zirconia particles, yttria particles) in the zirconia composition were measured for the average diameter of primary particles constituting the granules obtained through the preparation of the zirconia composition by individually using each raw material powder following the above methods. The measurement was conducted as follows.

Surface images (SEM images) were captured for the granular powder, using a scanning electron microscope (VE-9800 manufactured by Keyence under this trade name). The average primary particle diameter was calculated by image analysis after indicating grain boundaries on individual particles in the image.

The particle diameter was measured using image analysis software (Image-Pro Plus ver. 7.0.1 manufactured by Hakuto Co., Ltd. under this trade name). The captured SEM image was subjected to binarization, and the brightness range was adjusted to enhance clarity of the grain boundaries, allowing for the recognition of particles in the field (region).

The particle diameter from Image-Pro Plus represents the diameter through the center of gravity of the particle. The diameter through the center of gravity of the particle is the average of the measurements of the length of a line segment connecting the contour line and passing through the center of gravity determined from the contour line of the particle, conducted at 2-degree intervals with the center of gravity as the central point (the average of 180 particles).

The particle size measurement was conducted for particles not extending beyond the edges of the image. Note that particles not extending beyond the edges of the image are particles excluding those with contour lines extending beyond the screen of the SEM photographic image (particles with their contour lines interrupted by the boundary lines at the top, bottom, left, and right). To select the particle size of all particles not extending beyond the edges of the image, the option in Image-Pro Plus was used that excludes all particles lying on the boundary lines.

The particle size was determined for each granule in three fields for a sample from each Example and Comparative Example, and the result was averaged to find the average primary particle diameter.

<Stabilizer Content (mol%) in Zirconia Composition and Zirconia Pre-Sintered Body>

The stabilizer content (mol%) in the zirconia composition and zirconia pre-sintered body was measured as a stabilizer content relative to the total mole of zirconia and stabilizer, using an X-ray fluorescence (XRF) analyzer (RX3000, manufactured by Matsusada Precision Inc.).

<Evaluation of Crystal Phase Fractions of Zirconia Pre-Sintered Body>

The tetragonal fraction ft, cubic fraction $f_c$, and monoclinic fraction $f_m$ of the zirconia pre-sintered body were determined by analyzing its crystalline phases.

Specifically, the area intensities of the respective peaks (peak area intensities I) were determined through X-ray diffraction measurement conducted under the following conditions, using Automated Horizontal Multipurpose X-Ray Diffractometer (SmartLab, manufactured by Rigaku Corporation) and X-Ray Analysis Integrated Software (SmartLab Studio II, manufactured by Rigaku Corporation).

X-ray source: Cu K$\alpha$ ($\lambda$ = 1.54186 Å)
Goniometer length: 300 mm
Optical system: focusing technique
Detector: high-speed 1D X-ray detector (D/teX Ultra 250)
Monochromatization: K$\beta$ filter
Tube voltage: 40 kV
Tube current: 30 mA
Scan axis: 2$\theta$/$\theta$
Scan speed: 0.2°/min
Sampling step: 0.01 °

The crystal phase fractions were determined by assigning each peak to its corresponding crystalline phase, and calculating the respective fractions using the following formulae (2-1), (2-2), and (2-3).

$$\text{Tetragonal fraction } f_t \ (\%) = I_t \ / \ (I_m + I_t + I_c + I_y) \times 100 \qquad (2\text{-}1)$$

$$\text{Cubic fraction } f_c \ (\%) = I_c \ / \ (I_m + I_t + I_c + I_y) \times 100 \qquad (2\text{-}2)$$

$$\text{Monoclinic fraction } f_m \ (\%) = I_m \ / \ (I_m + I_t + I_c + I_y) \times 100 \qquad (2\text{-}3)$$

wherein $f_m$, ft, and $f_c$ represent the monoclinic fraction (%), tetragonal fraction (%), and cubic fraction (%), respectively, $I_m$ represents the peak area intensity near 2$\theta$ = 28.2°, where the peak top of the main peak of the monoclinic system appears in XRD measurement, It represents the peak area intensity near 2$\theta$ = 30.2°, where the peak top of the main peak of the tetragonal system appears in XRD measurement, $I_c$ represents the peak area intensity near $2\theta = 30.1°$, where the peak top of the main peak of the cubic system appears in XRD measurement, and $I_y$ represents the peak area intensity near $2\theta = 29.2°$, where the peak top of the main peak of undissolved yttria appears in XRD measurement.

For the measurements, the discotic zirconia pre-sintered bodies from each Example and Comparative Example were used as samples.

<Measurement of Undissolved Fraction of Yttria in Zirconia in Zirconia Pre-Sintered Body>

The undissolved yttria fraction $f_y$ in the zirconia pre-sintered body was determined through X-ray diffraction (XRD) measurement with Cu K$\alpha$ radiation, using the following formula (2-4).

$$f_y\ (\%) = I_y\ /\ (I_m + I_t + I_c + I_y) \times 100 \qquad (2\text{-}4)$$

wherein $f_y$ represents the fraction (%) of undissolved yttria, $I_m$ represents the peak area intensity near $2\theta = 28.2°$, where the peak top of the main peak of the monoclinic system appears in XRD measurement, It represents the peak area intensity near $2\theta = 30.2°$, where the peak top of the main peak of the tetragonal system appears in XRD measurement, $I_c$ represents the peak area intensity near $2\theta = 30.1°$, where the peak top of the main peak of the cubic system appears in XRD measurement, and $I_y$ represents the peak area intensity near $2\theta = 29.2°$, where the peak top of the main peak of undissolved yttria appears in XRD measurement.

<Measurement of Standard Deviation of Yttrium Element Distribution in Zirconia Pre-Sintered Body>

The yttrium element distribution of the zirconia pre-sintered body was measured under the following conditions, using a field-emission scanning electron microscope (FE-SEM Reglus 8220, manufactured by Hitachi High-Tech Corporation) and an energy dispersive X-ray analyzer (Aztec Energy X-Max 50, manufactured by Oxford Instruments). Yttrium elements were observed at 1.923 keV.

The standard deviation (mol%) of yttrium elements was determined for 10 particles originating from the stabilizer.

Measurement magnification: 20,000×
Analysis mode: point analysis
Acceleration voltage: 5 kV
Working distance: 15 mm $\pm$ 1 mm
X-ray extraction angle: 30 degrees
Dead time: 7%
Measurement time: 100 seconds

<Measurement Method for Average Primary Particle Diameter of Particles in Zirconia Pre-Sintered Body>

Surface images (SEM images) were captured for the zirconia pre-sintered body obtained in each Example or Comparative Example, using a scanning electron microscope (VE-9800 manufactured by Keyence under this trade name). The primary particle diameters of crystal particles were calculated by image analysis after indicating grain boundaries on individual crystal particles in the image.

The particle diameter was measured using image analysis software (Image-Pro Plus ver. 7.0.1 manufactured by Hakuto Co., Ltd. under this trade name). The captured SEM image was subjected to binarization, and the brightness range was adjusted to enhance clarity of the grain boundaries, allowing for the recognition of particles in the field (region).

The particle diameter from Image-Pro Plus represents the diameter through the center of gravity of the particle. The diameter through the center of gravity of the particle is the average of the measurements of the length of a line segment connecting the contour line and passing through the center of gravity determined from the contour line of the particle, conducted at 2-degree intervals with the center of gravity as the central point (the average of 180 particles).

The particle size measurement was conducted for particles not extending beyond the edges of the image. Note that particles not extending beyond the edges of the image are particles excluding those with contour lines extending beyond the screen of the SEM photographic image (particles with their contour lines interrupted by the boundary lines at the top, bottom, left, and right). To select the particle size of all particles not extending beyond the edges of the image, the option in Image-Pro Plus was used that excludes all particles lying on the boundary lines.

The particle size was determined for each crystal particle in three fields for a sample from each Example and Comparative Example, and the result was averaged to find the average primary particle diameter.

<Translucency Evaluation of Zirconia Sintered Body (Measurement of $\Delta L^*$(W-B))>

The pre-sintered bodies obtained in Examples and Comparative Examples were fired to fabricate zirconia sintered bodies (first sintered bodies) by setting the highest sintering temperature to 1,550°C with a hold time of 120 minutes at this temperature (120-minute sintering).

Subsequently, zirconia sintered bodies (second sintered bodies) were fabricated by firing pre-sintered bodies prepared in the same fashion, with the highest sintering temperature set at 1,550°C and a hold time of 10 minutes at this temperature (10-minute sintering).

The same rate of temperature increase and the same rate of temperature decrease were set for both 10-minute sintering and 120-minute sintering.

The two types of zirconia sintered bodies were ground into a 1.20 mm thick plate-shaped samples for translucency measurement. For these samples, the translucency was measured using a spectrophotometer (Crystaleye manufactured by Olympus Corporation under this trade name) in 7-band measurement mode with an LED light source.

Specifically, the plate-shaped zirconia sintered body samples were measured for translucency by measuring lightness (LW*) against a white background and lightness (LB*) against a black background for the same sample in chromaticity measurement, using the same measurement instrument in the same measurement mode with the same light source. The difference between these values ($\Delta L^* = $ (LW*) - (LB*)) was then determined as the translucency ($\Delta L^*$(W-B)) (the mean value for n = 3). The L* value is an L* value in the chromaticity (color space) of the L*a*b* color system (JIS Z 8781-4:2013).

The rate of change in translucency was then calculated as the ratio of $\Delta L_2^*$(W-B) to $\Delta L_1^*$(W-B) (the ratio $\Delta L_2^*$(W-B) / $\Delta L_1^*$(W-B)), where $\Delta L_1^*$(W-B) is a first translucency related to the first sintered body fabricated by 120-minute sintering at 1,550°C, and $\Delta L_2^*$(W-B) is a second translucency related to the second sintered body fabricated by 10-minute sintering at 1,550°C.

A rate of translucency change of 0.85 or more (85% or more) was considered acceptable.

The evaluation results for the zirconia pre-sintered bodies and zirconia sintered bodies are presented in Table 2.

EP 4 613 722 A1

[Table 1]

Zirconia composition

| | Monoclinic crystal 0Y | | Tetragonal crystal 3Y | | Cubic crystal 6Y | | Cubic crystal 10Y | | Cubic crystal 15Y | | Yttria | | Total yttria content [mol%] |
|---|---|---|---|---|---|---|---|---|---|---|---|---|---|
| | Mixing ratio [parts by mass] | Average particle diameter [nm] | Mixing ratio [parts by mass] | Average particle diameter [nm] | Mixing ratio [parts by mass] | Average particle diameter [nm] | Mixing ratio [parts by mass] | Average particle diameter [nm] | Mixing ratio [parts by mass] | Average particle diameter [nm] | Mixing ratio [parts by mass] | Average particle diameter [nm] | |
| Ex. 1 | | | 99.0 | 95 | | | | | | | 1.0 | 137 | 4.0 |
| Ex. 2 | | | 93.8 | 95 | | | | | | | 6.2 | 137 | 9.0 |
| Ex. 3 | 80.0 | 96 | | | | | 20.0 | 96 | | | | | 2.0 |
| Ex. 4 | 67.0 | 96 | | | 33.0 | 96 | | | | | | | 2.0 |
| Ex. 5 | 10.0 | 96 | | | | | 90.0 | 96 | | | | | 9.0 |
| Ex. 6 | 60.5 | 96 | 33.3 | 95 | | | | | | | 6.2 | 137 | 5.0 |
| Ex. 7 | 73.8 | 96 | 20.0 | 95 | | | | | | | 6.2 | 137 | 5.0 |
| Ex. 8 | 81.3 | 96 | | | 12.0 | 96 | | | | | 6.7 | 137 | 5.0 |
| Ex. 9 | 50.2 | 96 | | | 45.8 | 96 | | | | | 4.0 | 137 | 5.0 |
| Ex. 10 | | | 48.8 | 95 | 51.1 | 96 | | | | | 0.1 | 137 | 5.0 |
| Ex. 11 | | | 21.2 | 95 | | | 78.7 | 96 | | | 0.1 | 137 | 9.0 |
| Ex. 12 | | | 90.6 | 95 | 9.3 | 96 | | | | | 0.1 | 137 | 3.7 |
| Ex. 13 | 85.2 | 96 | | | | | | | 14.8 | 96 | | | 2.0 |
| Ex. 14 | 37.2 | 96 | | | | | | | 62.8 | 96 | | | 9.0 |
| Ex. 15 | | | 48.1 | 95 | | | | | 51.8 | 96 | 0.1 | 137 | 9.0 |
| Com. Ex. 1 | | | 66.7 | 95 | 33.3 | 96 | | | | | | | 4.0 |
| Com. Ex. 2 | | | | | 100.0 | 96 | | | | | | | 5.0 |
| Com. Ex. 3 | | | 100.0 | 95 | | | | | | | | | 3.0 |
| Com. Ex. 4 | 78.0 | 130 | 16.5 | 95 | | | | | | | 5.5 | 137 | 6.0 |

| | Zirconia composition | | | | | | | | | | | | |
|---|---|---|---|---|---|---|---|---|---|---|---|---|---|
| | Monoclinic crystal 0Y | | Tetragonal crystal 3Y | | Cubic crystal 6Y | | Cubic crystal 10Y | | Cubic crystal 15Y | | Yttria | | Total yttria content [mol%] |
| | Mixing ratio [parts by mass] | Average particle diameter [nm] | Mixing ratio [parts by mass] | Average particle diameter [nm] | Mixing ratio [parts by mass] | Average particle diameter [nm] | Mixing ratio [parts by mass] | Average particle diameter [nm] | Mixing ratio [parts by mass] | Average particle diameter [nm] | Mixing ratio [parts by mass] | Average particle diameter [nm] | |
| Com. Ex. 5 | | | 98.3 | 181 | | | | | | | 1.7 | 137 | 4.5 |
| Com. Ex. 6 | 78.0 | 96 | 16.5 | 95 | | | | | | | 5.5 | 400 | 6.0 |

[Table 2]

| | Zirconia pre-sintered body | | | | | | | Zirconia sintered body | | |
|---|---|---|---|---|---|---|---|---|---|---|
| | Monoclinic fraction | Tetragonal fraction | Cubic fraction | Undissolved yttria | Standard deviation of Y element distribution (mol%) | Average primary particle diameter (nm) | Total yttria content [mol%] | ΔL1*(W-B) | ΔL2*(W-B) | ΔL2*(W-B)/ΔL1*(W-B) |
| | fm [%] | ft [%] | fc [%] | fy [%] | | | | | | |
| Ex. 1 | | 96.2 | | 3.8 | 2.1 | 97.3 | 4.0 | 12.8 | 10.9 | 0.85 |
| Ex. 2 | | 90.1 | | 9.9 | 11.0 | 99.5 | 9.0 | 18.2 | 15.6 | 0.86 |
| Ex. 3 | 62.5 | | 37.5 | | 3.5 | 97.9 | 2.0 | 9.6 | 8.3 | 0.86 |
| Ex. 4 | 49.7 | | 50.3 | | 3.5 | 97.9 | 2.0 | 9.9 | 8.7 | 0.88 |
| Ex. 5 | 5.3 | | 94.7 | | 4.3 | 97.9 | 9.0 | 19.2 | 16.5 | 0.86 |
| Ex. 6 | 52.4 | 31.7 | | 15.9 | 12.7 | 100.2 | 5.0 | 16.1 | 14.7 | 0.91 |
| Ex. 7 | 63.9 | 26.0 | | 10.1 | 12.7 | 100.3 | 5.0 | 15.9 | 14.2 | 0.89 |
| Ex. 8 | 62.0 | | 27.5 | 10.5 | 12.7 | 100.7 | 5.0 | 15.0 | 12.9 | 0.86 |
| Ex. 9 | 34.6 | | 58.4 | 7.0 | 12.7 | 99.6 | 5.0 | 15.2 | 13.3 | 0.88 |
| ExP 10 | | 32.2 | 67.5 | 0.2 | 2.1 | 97.4 | 5.0 | 14.9 | 12.7 | 0.85 |
| Ex. 11 | | 11.8 | 88.0 | 0.2 | 8.3 | 110.1 | 9.0 | 18.0 | 15.4 | 0.86 |
| Exe 12 | | 82.8 | 17.0 | 0.2 | 3.3 | 96.3 | 3.7 | 12.3 | 10.5 | 0.85 |
| Ex. 13 | 75.6 | | 25.4 | | 3.8 | 98.5 | 2.0 | 9.8 | 8.6 | 0.88 |
| Ex. 14 | 23.4 | | 76.6 | | 4.5 | 98.5 | 9.0 | 19.4 | 17.0 | 0.88 |
| Ex. 15 | | 37.0 | 62.8 | 0.2 | 8.5 | 111.5 | 9.0 | 18.5 | 16.3 | 0.88 |
| Com. Ex. 1 | | 59.0 | 41.0 | | 1.5 | 97.2 | 4.0 | 14.9 | 12.1 | 0.81 |
| Com. Ex. 2 | | | 100.0 | | 0.2 | 97.9 | 5.0 | 15.4 | 11.5 | 0.75 |
| Com. Ex. 3 | 4.6 | 95.4 | | | 0.3 | 96.9 | 3.0 | 11.5 | 7.3 | 0.63 |
| Com. Ex. 4 | 83.9 | 6.9 | | 9.2 | 11.3 | 127.1 | 6.0 | 10.6 | 6.1 | 0.58 |
| Com. Ex. 5 | | 98.0 | | 2.0 | 0.8 | 180.2 | 4.5 | 14.3 | 10.1 | 0.71 |
| Com. Ex. 6 | 78.0 | 6.9 | | 15.1 | 21.2 | 113.7 | 6.0 | 9.8 | 6.4 | 0.65 |

36

As shown in Table 2, in Comparative Examples 1 to 6, the translucency was lower in short sintering with a retention time of 10 minutes at the highest sintering temperature, when compared to sintering with a 2-hour retention time at the same highest sintering temperature.

In contrast, in Examples 1 to 15, the translucency after short sintering with a 10-minute retention time at the highest sintering temperature was comparable to that achieved by sintering with a retention time of 2 hours at the same highest sintering temperature.

INDUSTRIAL APPLICABILITY

The zirconia pre-sintered bodies, compositions, and methods for their production according to the present invention are useful in dental material applications such as dental prostheses.

**Claims**

1. A zirconia pre-sintered body with zirconia particles having consolidated to an extent not reaching full sintering, the zirconia pre-sintered body comprising zirconia, and a stabilizer capable of preventing a phase transformation of zirconia, and satisfying the formula (1) below when $\Delta L_1^*(W\text{-}B)$ related to a first sintered body fabricated by sintering the zirconia pre-sintered body at 1,550°C for 120 minutes is compared to $\Delta L_2^*(W\text{-}B)$ related to a second sintered body fabricated by sintering the zirconia pre-sintered body at 1,550°C for 10 minutes,

$$\Delta L_2^*(W\text{-}B) \,/\, \Delta L_1^*(W\text{-}B) \geq 0.85 \qquad (1).$$

2. The zirconia pre-sintered body according to claim 1, which satisfies the following condition (i) or (ii),

   (i) the zirconia comprises tetragonal zirconia, and a part of the stabilizer is not dissolved in zirconia as a solid solution;
   (ii) the zirconia comprises monoclinic zirconia and cubic zirconia.

3. The zirconia pre-sintered body according to claim 1 or 2, wherein the stabilizer is yttria ($Y_2O_3$).

4. The zirconia pre-sintered body according to claim 3, which satisfies any of the following conditions (A-1), (A-2), (A-3), (A-4), (A-5), and (A-6),

   (A-1) the zirconia comprises monoclinic zirconia and tetragonal zirconia, where the monoclinic content is 55% or more and the tetragonal content is 10% or more, and a part of the yttria is not dissolved in zirconia as a solid solution;
   (A-2) the zirconia comprises tetragonal zirconia, and, without corresponding to (A-1), the tetragonal content is 10% or more, and a part of the yttria is not dissolved in zirconia as a solid solution;
   (A-3) the zirconia comprises monoclinic zirconia and cubic zirconia, where the monoclinic content is 55% or more, and the cubic content is 15% or more;
   (A-4) the zirconia comprises monoclinic zirconia and cubic zirconia, and, without corresponding to (A-3), the cubic content is 15% or more;
   (A-5) the zirconia comprises tetragonal zirconia and cubic zirconia, where the tetragonal content is 5% or more and less than 50%, and the cubic content is 50% or more and less than 95%, and a part of the yttria is not dissolved in zirconia as a solid solution;
   (A-6) the zirconia comprises tetragonal zirconia and cubic zirconia, and, without corresponding to (A-5), the cubic content is 10% or more and less than 50%, and a part of the yttria is not dissolved in zirconia as a solid solution,
   the tetragonal content, cubic content, and monoclinic content in the above (A-1), (A-2), (A-3), (A-4), (A-5), or (A-6) being calculated from the following formulae, respectively,

   $$\text{tetragonal fraction } f_t\ (\%) = I_t \,/\, (I_m + I_t + I_c + I_y) \times 100 \qquad (2\text{-}1)$$

   $$\text{cubic fraction } f_c\ (\%) = I_c \,/\, (I_m + I_t + I_c + I_y) \times 100 \qquad (2\text{-}2)$$

$$\text{monoclinic fraction } f_m \text{ (\%)} = I_m / (I_m + I_t + I_c + I_y) \times 100 \quad (2\text{-}3),$$

wherein $f_m$, ft, and $f_c$ represent the monoclinic fraction (%), tetragonal fraction (%), and cubic fraction (%), respectively, $I_m$ represents the peak area intensity near $2\theta = 28.2°$, where the peak top of the main peak of the monoclinic system appears in XRD measurement, It represents the peak area intensity near $2\theta = 30.2°$, where the peak top of the main peak of the tetragonal system appears in XRD measurement, $I_c$ represents the peak area intensity near $2\theta = 30.1°$, where the peak top of the main peak of the cubic system appears in XRD measurement, and $I_y$ represents the peak area intensity near $2\theta = 29.2°$, where the peak top of the main peak of undissolved yttria appears in XRD measurement.

5. The zirconia pre-sintered body according to claim 3 or 4, wherein the standard deviation of yttrium element distribution is 2 mol% or more and less than 21 mol%.

6. The zirconia pre-sintered body according to claim 4 or 5, which satisfies the condition (A-1) or (A-2), and in which the proportion of yttria not dissolved in zirconia as a solid solution is 1 to 25%.

7. The zirconia pre-sintered body according to claim 4 or 5, which satisfies the condition (A-3) or (A-4), and in which a part of the yttria is not dissolved in zirconia as a solid solution, and the proportion of the yttria not dissolved in zirconia as a solid solution is 1 to 15%.

8. The zirconia pre-sintered body according to any one of claims 1 to 7, wherein the content of the stabilizer is 2 to 9 mol% relative to the total mole of the zirconia and the stabilizer.

9. The zirconia pre-sintered body according to any one of claims 1 to 8, which has a density of $3.6 \text{ g/cm}^3$ or less.

10. The zirconia pre-sintered body according to any one of claims 1 to 9, which has an average primary particle diameter of 40 to 110 nm.

11. A zirconia composition comprising zirconia, and a stabilizer capable of preventing a phase transformation of zirconia, and satisfying the formula (1) below when $\Delta L_1{}^*(\text{W-B})$ related to a first sintered body fabricated by sintering the zirconia composition at 1,550°C for 120 minutes is compared to $\Delta L_2{}^*(\text{W-B})$ related to a second sintered body fabricated by sintering the zirconia composition at 1,550°C for 10 minutes,

$$\Delta L_2{}^*(\text{W-B}) / \Delta L_1{}^*(\text{W-B}) \geq 0.85 \quad (1).$$

12. The zirconia composition according to claim 11, which satisfies the following condition (i) or (ii),

(i) the zirconia comprises a tetragonal zirconia powder (T), and a part of the stabilizer is a stabilizer powder that is not dissolved in zirconia as a solid solution;
(ii) the zirconia comprises a monoclinic zirconia powder (M) and a cubic zirconia powder (C).

13. The zirconia composition according to claim 12, which satisfies the condition (i), and further comprises a monoclinic zirconia powder (M).

14. The zirconia composition according to claim 13, wherein the ratio of the total mass of the zirconia powder (T) and the zirconia powder (M) to the mass of the stabilizer powder is 85.0 mass%:15.0 mass% to 99.8 mass%:0.2 mass%.

15. The zirconia composition according to claim 13 or 14, wherein the zirconia powder (M) is 0 to 85 mass% of the total mass of the zirconia powder (T) and the zirconia powder (M).

16. The zirconia composition according to any one of claims 12 to 15, wherein the zirconia powder (T) comprises a dissolved stabilizer capable of preventing a phase transformation of zirconia, and the content of the dissolved stabilizer is 2 to 4 mol% relative to the total mole of the zirconia and the stabilizer.

17. The zirconia composition according to claim 12, which satisfies the condition (ii), and in which a part of the stabilizer is not dissolved in zirconia as a solid solution.

18. The zirconia composition according to claim 12 or 17, wherein the ratio of the total mass of the zirconia powder (M) and the zirconia powder (C) to the mass of the stabilizer powder is 85.0 mass%:15.0 mass% to 100 mass%:0 mass%.

19. The zirconia composition according to claim 12, 17, or 18, wherein the zirconia powder (C) is 15.0 to 95.0 mass% of the total mass of the zirconia powder (M) and the zirconia powder (C).

20. The zirconia composition according to any one of claims 12, 17 to 19, wherein the content of the stabilizer in the zirconia powder (M) is 0 to 1 mol% relative to the total mole of the zirconia powder (M) and the stabilizer.

21. The zirconia composition according to any one of claims 12, 17 to 20, wherein the zirconia powder (C) comprises a dissolved stabilizer capable of preventing a phase transformation of zirconia, and the content of the dissolved stabilizer is 5 mol% or more and 15 mol% or less relative to the total mole of the zirconia powder (C) and the stabilizer.

22. The zirconia composition according to claim 12, which satisfies the condition (i), and further comprises a cubic zirconia powder (T), wherein the ratio of the total mass of the zirconia powder (T) and the zirconia powder (C) to the mass of the stabilizer powder is 88.0 mass%:12.0 mass% to 99.999 mass%:0.001 mass%.

23. The zirconia composition according to any one of claims 11 to 22, wherein the zirconia powder (T), the zirconia powder (C), and the zirconia powder (M) each have an average primary particle diameter (r1) of 40 to 110 nm.

24. A method for producing a zirconia pre-sintered body, comprising firing the zirconia compositions of claims 11 to 23 to such an extent that zirconia particles do not fully sinter.

25. A method for producing a zirconia sintered body, comprising sintering the zirconia pre-sintered bodies of claims 1 to 10.

**INTERNATIONAL SEARCH REPORT**

| International application No. |
| --- |
| **PCT/JP2023/039628** |

| A. | CLASSIFICATION OF SUBJECT MATTER |
| --- | --- |

*C04B 35/486*(2006.01)i; *A61C 13/083*(2006.01)i; *A61K 6/818*(2020.01)i; *A61K 6/824*(2020.01)i; *A61L 27/10*(2006.01)i; *C01G 25/00*(2006.01)i; *C01G 25/02*(2006.01)i
FI:  C04B35/486; C01G25/02; A61L27/10; A61K6/818; C01G25/00; A61C13/083; A61K6/824

According to International Patent Classification (IPC) or to both national classification and IPC

| B. | FIELDS SEARCHED |
| --- | --- |

Minimum documentation searched (classification system followed by classification symbols)

   C04B35/486; A61C13/083; A61K6/818; A61K6/824; A61L27/10; C01G25/00; C01G25/02

Documentation searched other than minimum documentation to the extent that such documents are included in the fields searched

   Published examined utility model applications of Japan 1922-1996
   Published unexamined utility model applications of Japan 1971-2023
   Registered utility model specifications of Japan 1996-2023
   Published registered utility model applications of Japan 1994-2023

Electronic data base consulted during the international search (name of data base and, where practicable, search terms used)

| C. | DOCUMENTS CONSIDERED TO BE RELEVANT | | |
| --- | --- | --- | --- |
| Category* | Citation of document, with indication, where appropriate, of the relevant passages | | Relevant to claim No. |
| X | JP 2022-162005 A (KURARAY NORITAKE DENTAL INC.) 21 October 2022 (2022-10-21) paragraphs [0011], [0012], [0136]-[0153] | | 1-16, 18-19, 22-25 |
| A | | | 17, 20-21 |
| A | WO 2020/179876 A1 (KURARAY NORITAKE DENTAL INC.) 10 September 2020 (2020-09-10) entire text | | 1-25 |
| A | WO 2020/179877 A1 (KURARAY NORITAKE DENTAL INC.) 10 September 2020 (2020-09-10) entire text | | 1-25 |
| P, X | WO 2023/120674 A1 (KURARAY NORITAKE DENTAL INC.) 29 June 2023 (2023-06-29) paragraphs [0147]-[0185] | | 1-8, 10-11, 24-25 |
| P, A | | | 9, 12-23 |

| ☐ Further documents are listed in the continuation of Box C. | ☑ See patent family annex. |
| --- | --- |

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| **20 December 2023** | **09 January 2024** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

## INTERNATIONAL SEARCH REPORT
### Information on patent family members

International application No.

**PCT/JP2023/039628**

| Patent document cited in search report | | | Publication date (day/month/year) | Patent family member(s) | | | Publication date (day/month/year) |
|---|---|---|---|---|---|---|---|
| JP | 2022-162005 | A | 21 October 2022 | US 2020/0317581 A1 paragraphs [0011]-[0013], [0086], [0087], [0163]-[0176] WO 2018/056330 A1 EP 3517518 A1 CN 109689593 A KR 10-2019-0047701 A | | | |
| WO | 2020/179876 | A1 | 10 September 2020 | US 2022/0153649 A1 entire text EP 3936490 A1 CN 113490653 A KR 10-2021-0129198 A | | | |
| WO | 2020/179877 | A1 | 10 September 2020 | US 2022/0135486 A1 entire text EP 3936081 A1 CN 113490468 A KR 10-2021-0133275 A | | | |
| WO | 2023/120674 | A1 | 29 June 2023 | (Family: none) | | | |

Form PCT/ISA/210 (patent family annex) (January 2015)

41

**REFERENCES CITED IN THE DESCRIPTION**

**Patent documents cited in the description**

- JP 2018052806 A
- WO 2018056330 A
- JP 2020033338 A
- JP 6543926 B
- WO 2014126034 A
- JP 2000159621 A